# EUROPEAN PATENT SPECIFICATION

(11) **EP 4 010 043 B1**
(45) Date of publication and mention of the grant of the patent: **30.08.2023**
(21) Application number: 20754364.6
(22) Date of filing: 13.07.2020
(51) Int. Cl.: A61M 60/17, A61M 60/268, A61M 60/295, A61M 60/427, A61M 60/497, A61M 60/837, A61M 60/843, A61M 60/861, A61M 60/869, A61M 60/894

(54) **CARDIAC CHAMBER PROSTHESIS AND RELATED CARDIAC ASSISTANCE SYSTEM**
HERZKAMMERPROTHESE UND ENTSPRECHENDES HERZUNTERSTÜTZUNGSSYSTEM
PROTHÈSE DE CHAMBRE CARDIAQUE ET SYSTÈME D'ASSISTANCE CARDIAQUE ASSOCIÉ

(30) Priority: 12.07.2019 IT 201900011640
(43) Date of publication of application: 15.06.2022
(73) Proprietor: Romano, Salvatore, 50133 Firenze (FI) (IT)
(72) Inventor: Romano, Salvatore, 50133 Firenze (FI) (IT)
(74) Representative: IP Sextant s.r.l.
(86) International application number: PCT/IB2020/056544
(87) International publication number: WO 2021/009651

(56) References cited:
- US-A- 5 139 517
- US-A1- 2005 246 013
- US-A1- 2011 153 010
- US-A1- 2017 136 162
- US-B1- 7 341 584

## Description

The present invention relates to a cardiac chamber prosthesis, selected from the group consisting of left ventricle, left atrium, right ventricle and right atrium, that is capable, in a simple, adaptable, efficient and reliable way, to have a physiological morphology and to operate according to an operation that most conforms to its physiological characteristics. The present invention further relates to an assembly of cardiac chamber prostheses having two or more of such cardiac chamber prostheses and to a cardiac assistance system provided with such a cardiac chamber prosthesis or such assembly of cardiac chamber prostheses. Also, the cardiac chamber prosthesis according to the invention has reduced size and weight, making it easy to implant and control in a cardiac assistance system requiring reduced energy consumption, significantly increasing the therapeutic options for patients with severe heart failure obtaining better therapeutic results. Furthermore, the cardiac chamber prosthesis, and the related cardiac assistance system, according to the invention are simple and inexpensive to manufacture.

As known, the heart is a muscle that pumps blood through the body: the right side of the heart receives deoxygenated blood from the body and pumps it to the lungs, where the blood is enriched with oxygen and carbon dioxide is removed from it, while the left side of the heart receives oxygenated blood that returns from the lungs and pumps it to the body, where it supplies the cells with oxygen and nutrients.

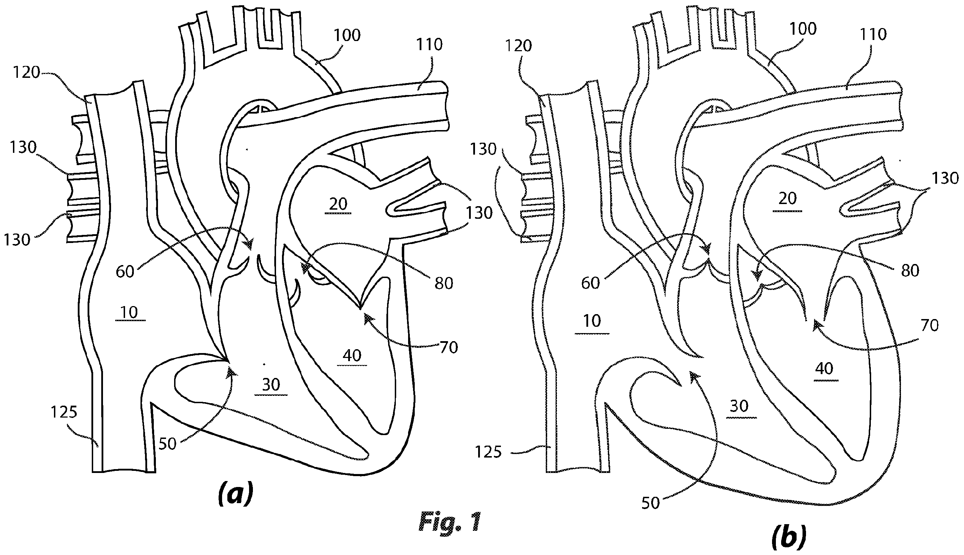

With reference to Figure 1, it can be seen that the heart has four cardiac chambers, i.e. two upper atria and two lower ventricles, namely: the right atrium 10, the left atrium 20, the right ventricle 30 and the left ventricle 40. The atria 10 and 30 are the receiving cardiac chambers and the ventricles 30 and 40 are the pumping cardiac chambers.

Also, the heart has four heart valves, namely: the tricuspid valve 50 interposed between the right atrium 10 and the right ventricle 30; the pulmonary valve 60 interposed between the right ventricle 30 and the pulmonary artery 110; the mitral valve 70 interposed between the left atrium 20 and the left ventricle 40; and the aortic valve 80 interposed between the left ventricle 40 and the aorta 100. The function of the heart valves is to ensure that the blood flows in the correct direction through the heart (as indicated by the dotted arrows). In particular, deoxygenated blood is pumped from the right ventricle 30 into the pulmonary artery 110 and, from this, into the lungs. Similarly, oxygenated blood is pumped from the left ventricle 40 into the aorta 100 and, from this, through the body. Moreover, the superior vena cava 120 and the inferior vena cava 125 carry deoxygenated blood from the upper half and lower half of the body, respectively, to the right atrium 10, while the pulmonary veins 130 carry oxygenated blood from the lungs to the left atrium 20.

In greater detail, during the systolic phase of a cardiac chamber, the heart assumes a configuration of muscle contraction in which the cardiac chamber under consideration contracts and empties of blood. In atrial systole (shown in Fig. 1b), only the atria 10 and 20 contract, the tricuspid valve 50 and the mitral valve 70 are open while the pulmonary valve 60 and the aortic valve 80 are closed, and blood is transferred from the atria 10 and 20 to the ventricles 30 and 40, respectively; in ventricular systole (shown in Fig.1a) only the ventricles 30 and 40 contract, the tricuspid valve 50 and the mitral valve 70 are closed and the pulmonary valve 60 and the aortic valve 80 are open, and blood is transferred from the ventricles 30 and 40 to the pulmonary artery 110 and aorta 100, respectively.

Similarly, during the diastolic phase of a cardiac chamber, the heart assumes a configuration of muscle relaxation in which the cardiac chamber under consideration widens and fills with blood. In atrial diastole (shown in Fig.1a) the atria 10 and 20 expand (earlier than an atrial systole), the tricuspid valve 50 and the mitral valve 70 are closed, and the blood from the upper and lower venae cavae 120 and 125 and from the pulmonary veins 130 fills the atria 10 and 20, respectively; in ventricular diastole (shown in Fig.1b) ventricles 30 and 40 expand (earlier than a ventricular systole) until they reach the maximum volume, also known as ventricular telediastolic volume, the pulmonary valve 60 and aortic valve 80 are closed, and the blood from the atria 10 and 20 fills the ventricles 30 and 40, respectively. In particular, the atrial systole occurs in correspondence with the ventricular diastole and the atrial diastole occurs in correspondence with the ventricular systole.

In other words, atrial and ventricular systoles and atrial and ventricular diastoles in the sinus rhythm (i.e. in the physiology of a healthy person) are synchronized according to an accurate timing that allows the heart to reoxygenate the deoxygenated blood coming from the body by pumping it towards the lungs and, subsequently, through the body.

Some diseases of the heart can decrease its pumping power. When the heart does not pump blood properly, the fluid can accumulate in the lungs and other parts of the body. Symptoms of heart failure generally derive from the heart inability to supply an adequate blood flow to the vital organs of the body. In this case, a cardiac assistance system may be necessary when drugs and other surgical options are not sufficient to resolve heart failure.

Among the prior art cardiac assistance systems, a widely used solution is the axial flow ventricular assist device (VAD), which includes a mechanical pump that has significantly improved therapeutic options for patients with severe heart failure. Such VAD devices, as for instance the Jarvik device available from the US company Jarvik Heart, Inc. and the DeBakey device available from the US company MicroMed Technology, Inc., are described by documents WO 89/07427 A1, WO 99/49912 A1, US 2018/0303991 A1 and WO 2018/078370 A1 and by Ootaki Y. et al. in "Phasic coronary blood flow pattern during a continuous flow left ventricular assist support", Eur. J. of Cardio-thorac. Surg. 28 (2005) 711-16, by Radovancevic B. et al. in "End-organ Function in Patients on Long-term Circulatory Support With Continuous- or Pulsatile-flow Assist Devices", J. of Heart and Lung Transp. 2007, 815-18, by Frazier O.H. et al. in "Multicenter clinical evaluation of the HeartMate vented electric left ventricular assist system in patients awaiting heart transplantation", J. Thorac. Cardiovasc. Surg. 2001, 122:1186-95, by Letsou G.V. et al. in "Continuous axial flow left ventricular assist device (Jarvik 2000) maintains kidney and liver perfusion for up to 6 months", Ann. Thorac. Surg. 2003; 76:1167-70, by Thalmann M. et al. in "Physiology of continuous blood flow in recipients of rotary cardiac assist devices", J. Heart Lung Transplant 2005; 24:237-45, by Thohan V. et al. in "Cellular and hemodynamics responses of failing myocardium to continuous flow mechanical circulatory support using the DeBakey - Noon left ventricular assist device: a comparative analysis with pulsatile-type devices", J. Heart Lung Transplant 2005; 24:566-75, and by Hetzer R. et al. in "First experiences with a novel magnetically suspended axial flow left ventricular assist device", Eur. J. Cardiothorac. Surg. 2004; 25:964-70.

VAD devices are based on rotary pumps developed to address some problems of previous pulsatile devices, such as size and complexity, implantation difficulties and adverse events (e.g., perioperative bleeding, infection, thrombosis and device malfunction). The miniaturized implantable pumps of the VAD devices provide a continuous flow, instead of a pulsatile one, by using an electromagnetic mechanism consisting of a rotor with impeller blades. Current non-pulsatile axial flow pumps tend to have a compact design, use a single moving part, and have lower energy requirements. For instance, the Jarvik device is configured to be implanted in the body of the left ventricle and therefore it does not need an inflow cannula. Long-term implants of rotary VAD devices for left ventricle are being increasingly used for the treatment of patients with end-stage heart failure.

However, currently available VAD devices also suffer from some drawbacks.

First of all, the rotary pumps with which they are provided operate at a speed that must be carefully adjusted to avoid both backflow and excessive suction. In fact, backflow (regurgitation) could occur when the pump speed (rpm) is set at a too low value, causing blood to return from the aorta 100 to the left ventricle 40 through the pump, while excessive suction could occur when the pump tries to draw more blood than what available in the left ventricle 40 due to the high speed of the pump. Suction could cause ventricular collapse of the left ventricle 40, that can cause chest pain and damage to heart tissue. Consequently, the pump flow, that mainly depends on speed, that depend on heart rate, and that is not adjustable in a pulsatile manner, is not capable to physiologically conform to the sinus rhythm and cannot follow peripheral circulation changes with a resulting lack of adaptation of cardiac output during use. Also, the implantation of such device is extremely invasive and requires an irreversible damage of the cardiac chamber in which it is implanted and further stresses the other cardiac chambers stressed by an unnatural heart flow.

The outflow line from the pump is connected to the descending aorta. At high pump speed, the aortic valve 80 remains closed and the VAD device completely replaces the ventricular cardiac function. To limit the possible formation of thrombus in the aortic valve 80, current VAD systems reduce the speed for 10 seconds per minute, and during this interval the aortic valve 80 opens again and pulsatile flow is appreciable. As the number of revolutions of the rotary pump increases again, the pulsatility of the aortic flow decreases, but it may not completely disappear thanks to a minimum pressure sufficient to continue to open the aortic valve 80. When the aortic valve 80 returns to remain closed, the pressure gradient between the left ventricle 40 and aorta 100 actually changes with the cardiac cycle because the changes in pressure, caused by the opening and closing of the mitral valve 70 (and to a much lesser extent by the residual ventricular systole), are transmitted into the aorta 100 through the pump. The study of the arterial pulse profile allows to appreciate the dicrotic notch in the descending part of the pressure curve that is correlated to the closure of the mitral valve 70 (the aortic valve 60 is constantly closed in this period of 50 seconds). In other words, movements of a myocardium, in which a VAD device that produces a continuous flow is implanted, contribute to a residual slight pulsatility of the resulting flow of the circulatory system.

In conclusion, VAD devices produce a non-pulsatile and, consequently, non-physiological flow (i.e. not conforming to the sinus rhythm) and their hemodynamic characteristics are different from the pulsatile devices.

As stated above, at a given rotational speed, the flow through a rotary pump is continuous, instead of pulsatile as in a physiological flow.

Also, if the pump speed is too high, the variations typical of the morphology of the waveform of the native arterial pressure tend to disappear and the dicrotic notch is absent (indicating that the aortic valve 60 is closed).

Finally, with a VAD device having a rotary pump, fluctuations in left ventricular pressure are transmitted to the arteries through the VAD device even when the pump speed is high enough to keep the aortic valve 60 closed. This can have important clinical consequences ( e.g. fusion of the cusp of the aortic valve 60 and thrombosis in the ascending aorta) and current research is aimed at optimizing their use to prevent such complications.

To overcome these drawbacks, other devices have been developed that enclose the entire organ, such as those described in documents US 2016/0317729 A1 and US 2016/0346085 A1. An intraventricular orthotopic pump used in combination with the natural heart is described in US 5139517 A.

However, the latter devices are equally highly invasive and non-selective of the heart chambers, which consequently can be deteriorated and damaged over time, and are not capable to conform to a physiological morphology and can entail problems of electrophysiological nature.

It is an object of this invention, therefore, to selectively assist the operation of any cardiac chamber, and not only the left ventricle, in a simple, adaptable, efficient and reliable way, so as to ensure that the cardiac chamber has a physiological morphology and an operation that most conforms to its physiological characteristics.

It is specific subject matter of the present invention as defined by independent claim 1 a cardiac chamber prosthesis configured to be implanted in a cardiac chamber of a patient selected from the group consisting of left ventricle, left atrium, right ventricle and right atrium,
wherein the cardiac chamber comprises a native outlet valve and at least one inlet aperture selected from the group comprising a native inlet valve and one or more outlet mouths of venae cavae or pulmonary veins,
wherein the cardiac chamber prosthesis comprises:
   - an inner elastic membrane, having an inner surface delimiting an elastically variable volume, provided with a first aperture delimited by a respective first border and with at least one second aperture delimited by a respective second border,
   - a reference support elastic membrane structure comprising or consisting of a outer elastic membrane provided with a first aperture delimited by a respective first border and with at least one second aperture delimited by a respective second border, wherein the outer elastic membrane is further provided with a plurality of clips configured to grip an inner wall of the cardiac chamber,
wherein the borders of said first apertures of the inner and outer elastic membranes are integrally coupled to each other to form an outlet border configured to surround and be sutured on the native outlet valve,
wherein the borders of said second aperture of the inner and outer elastic membranes are integrally coupled to each other to form at least one inlet border configured to surround and be sutured on said at least one inlet aperture,
wherein the inner elastic membrane and the reference support elastic membrane structure are connected to each other by means of a plurality of primary variable connection elements,
whereby the inner elastic membrane and the reference support elastic membrane structure delimit a primary interspace between them that is configured to receive a fluid with varying amount and/or pressure so as to dynamically modify a volume of the primary interspace and said elastically variable volume delimited by the inner surface of the inner elastic membrane.

According to another aspect of the invention, the outer elastic membrane can have a coefficient of elasticity not greater, optionally lower, than a coefficient of elasticity of the inner elastic membrane.

According to a further aspect of the invention, each one of the inner and outer elastic membranes can have a variable coefficient of elasticity, wherein each one of the inner and outer elastic membranes optionally comprises two or more areas having respective coefficients of elasticity.

According to an additional aspect of the invention, said fluid can be a liquid, optionally a haemocompatible solution, more optionally sterile saline solution, or a gas, optionally helium.

According to another aspect of the invention, the cardiac chamber prosthesis can further comprise at least one artificial valve integrally coupled to the outlet border or said at least one inlet border.

According to a further aspect of the invention, said primary variable connection elements can be primary elastic ties each one of which has a coefficient of elasticity greater than a coefficient of elasticity of the outer elastic membrane and than a coefficient of elasticity of the inner elastic membrane, wherein said primary elastic ties have coefficients of elasticity optionally variable depending on the area of the inner elastic membrane to which they are connected.

According to an additional aspect of the invention, the cardiac chamber prosthesis can be configured to be contained inside a catheter.

According to another aspect of the invention, the outer elastic membrane and/or the inner elastic membrane can comprise portions which are radiopaque.

According to a further aspect of the invention, the primary interspace can be configured to be in fluid communication with at least one variable volume elastic bag with which the prosthesis is provided or with an external pump.

According to an additional aspect of the invention, at least one between the inner elastic membrane and the outer elastic membrane can have a discontinuous structure, optionally selected from the group comprising:
- a honeycomb discontinuous structure formed by a plurality of elastic plates, more optionally hexagonal ones, connected to each other along elastic junction lines, wherein more optionally the elastic junction lines have a coefficient of elasticity greater than a coefficient of elasticity of the elastic plates, and
- a discontinuous structure formed by a plurality of elastic polygonal plates, more optionally with pentagonal and/or hexagonal shape, connected to each other along elastic junction lines, wherein more optionally said elastic junction lines have a coefficient of elasticity greater than a coefficient of elasticity of said elastic polygonal plates.

According to another aspect of the invention, the inner elastic membrane and the outer elastic membrane can be each provided with a plurality of electrodes configured to electrically and/or electromagnetically polarise the inner elastic membrane and the outer elastic membrane, respectively, under control of an electronic processing unit connected to said electrodes through respective control cables.

According to a further aspect of the invention, the reference support elastic membrane structure can further comprise an intermediate elastic membrane that is interposed between the outer elastic membrane and the inner elastic membrane, wherein the intermediate elastic membrane optionally has coefficient of elasticity not grater, more optionally lower, than a coefficient of elasticity of the outer elastic membrane and than a coefficient of elasticity of the inner elastic membrane,
wherein the intermediate elastic membrane is provided with a first aperture delimited by a respective first border, integrally coupled to the borders of said first apertures of the inner and outer elastic membranes, and with at least one second aperture delimited by a respective second border, integrally coupled to the borders of said second apertures of the inner and outer elastic membranes,
wherein the intermediate elastic membrane and the outer elastic membrane are connected to each other by means of a plurality of secondary variable connection elements, optionally consisting of secondary elastic ties each one of which more optionally has a coefficient of elasticity greater than the coefficient of elasticity of the outer elastic membrane and than the coefficient of elasticity of the intermediate elastic membrane, wherein said secondary elastic ties have coefficients of elasticity variable depending on the area of the intermediate elastic membrane to which they are connected,
whereby said plurality of primary variable connection elements connect the inner elastic membrane and the intermediate elastic membrane to each other,
whereby the outer elastic membrane and the intermediate elastic membrane delimit a secondary interspace between them that is configured to receive a fluid, optionally consisting in a liquid, more optionally a haemocompatible solution, still more optionally sterile saline solution, or in a gas, optionally helium.

According to an additional aspect of the invention, the secondary interspace can be configured to receive said fluid with varying amount and/or pressure, so as to dynamically modify a volume of the secondary interspace, wherein the secondary interspace is optionally configured to be in fluid communication with at least one variable volume elastic bag with which the prosthesis is provided or with an external pump.

According to another aspect of the invention, the intermediate elastic membrane can have a discontinuous structure, optionally selected from the group comprising:
- a honeycomb discontinuous structure formed by a plurality of elastic plates, more optionally hexagonal ones, connected to each other along elastic junction lines, wherein more optionally said elastic junction lines have a coefficient of elasticity greater than a coefficient of elasticity of said elastic plates, and
- a discontinuous structure formed by a plurality of elastic polygonal plates, more optionally with pentagonal and/or hexagonal shape, connected to each other along elastic junction lines, wherein more optionally said elastic junction lines have a coefficient of elasticity greater than a coefficient of elasticity of said elastic polygonal plates.

According to a further aspect of the invention, the intermediate elastic membrane can be provided with a plurality of electrodes configured to electrically and/or electromagnetically polarise the intermediate elastic membrane under control of said electronic processing unit connected to said electrodes through respective one or more control cables.

According to an additional aspect of the invention, the cardiac chamber prosthesis can be configured to be implanted:
- in a left ventricle, whereby the outlet border is an aortic border configured to surround and be sutured on a native aortic valve and said at least one inlet border consists of a mitral border configured to surround and be sutured on a native mitral valve, or
- in a left atrium, whereby the outlet border is a mitral border configured to surround and be sutured on a native mitral valve and said at least one inlet border consists of one or more borders of pulmonary veins configured to surround and be sutured in correspondence of respective one or more outlet mouths of pulmonary veins, or
- in a right ventricle, whereby the outlet border is a pulmonary valve border configured to surround and be sutured on a native pulmonary valve and said at least one inlet border consists of a tricuspid valve border configured to surround and be sutured on a native tricuspid valve, or
- in a right atrium, whereby the outlet border is a tricuspid valve border configured to surround and be sutured on a native tricuspid valve and said at least one inlet border consists of one or more borders of venae cavae configured to surround and be sutured in correspondence of respective one or more outlet mouths of venae cavae.

It is also specific subject matter of the present invention an assembly of cardiac chamber prostheses comprising two to four cardiac chamber prostheses as previously described, wherein said cardiac chamber prostheses are configured to be implanted in respective two or more cardiac chambers of a patient.

According to another aspect of the invention, said cardiac chamber prostheses can comprise or consist of two cardiac chamber prostheses associated to each other which are configured to be implanted in a left ventricle and in a left atrium, or in a right ventricle and in a right atrium, wherein the primary interspaces of the associated two cardiac chamber prostheses are in fluid communication with each other through a communication duct, whereby the volume of the primary interspace of one of the associated two cardiac chamber prostheses is configured to increase when the volume of the primary interspace of the other one of the associated two cardiac chamber prostheses decreases, and vice versa, through a displacement of fluid between the primary interspaces of the associated two cardiac chamber prostheses.

It is still specific subject matter of the present invention a cardiac assistance system comprising:
- a cardiac chamber prosthesis or an assembly of cardiac chamber prostheses as previously described,
- at least one pump and/or an electronic processing unit configured to control an amount and/or a pressure of said fluid received in the primary interspace of said cardiac chamber prosthesis or of the cardiac chamber prostheses of said assembly of cardiac chamber prostheses so as to dynamically modify the volume of the primary interspace of said cardiac chamber prosthesis or of the cardiac chamber prostheses of said assembly of cardiac chamber prostheses.

The cardiac chamber prosthesis according to the invention, as well as the related assembly, allows to assist the operation of any cardiac chamber (or of two or more cardiac chambers) in a physiological way also adaptable to different circumstances thanks to the electrical and/or electromagnetic and/or pneumatic control.

In particular, the cardiac chamber prosthesis, the related assembly and the related cardiac assistance system, according to the invention, significantly improve the therapeutic options for patients with severe heart failure. In fact, the cardiac assistance system according to the invention is capable to produce a pulsatile and physiological flow the hemodynamic characteristics of which are different from the continuous ones obtainable with a VAD device based on a rotary pump. At a given activation speed, the cardiac chamber prosthesis according to the invention, as well as the related assembly, operates in a variable and adaptable way and it is sensitive to the pressure gradient that passes through it (e.g., in case of application to the left ventricle 40, this gradient is the one between the pressure in the aorta 100 and the pressure in the left ventricle 40 during the ventricular systole while it is the one between the pressure in the left atrium 20 and the pressure in the left ventricle 40 during the atrial systole). Also, the waveform of the obtained arterial pressure conforms to the physiological one of the human heart in which the dicrotic notch is present (i.e. the aortic valve 80 closes).

Furthermore, the cardiac chamber prosthesis, the related assembly and the related cardiac assistance system according to the invention have reduced size and weight, being easy to implant and control and requiring reduced energy consumption.

Finally, the cardiac chamber prosthesis, the related assembly and the related cardiac assistance system according to the invention are simple and inexpensive to make.

The present invention will be now described, by way of illustration and not by way of limitation, according to its preferred embodiments, by particularly referring to the Figures of the annexed drawings, in which:
Figure 1 shows a schematic sectional view of a heart;
Figure 2 shows a schematic perspective view of a first embodiment of the prosthesis according to the invention, from which a part has been removed;
Figure 3 shows a schematic sectional view of a heart in which the prosthesis of Figure 2 has been implanted;
Figure 4 shows a schematic front view of a patient the heart in Figure 3 of which is visible;
Figure 5 shows a schematic perspective view of a second embodiment of the prosthesis according to the invention, from which a part has been removed (Fig. 5a), and a perspective view of an enlarged portion thereof (Fig. 5b);
Figure 6 shows a schematic sectional view of a heart in which a third embodiment of the prosthesis according to the invention has been implanted;
Figure 7 shows a schematic front view of a patient the heart in Figure 6 of which is visible;
Figure 8 shows a schematic sectional view of a heart in which a fourth embodiment of the prosthesis according to the invention has been implanted;
Figure 9 shows a schematic sectional view of a heart in which a first embodiment of the assembly of cardiac chamber prostheses according to the invention has been implanted;
Figure 10 shows a schematic sectional view of a heart in which a second embodiment of the assembly of cardiac chamber prostheses according to the invention has been implanted;
Figure 11 shows a schematic sectional view of a heart in which a third embodiment of the assembly of cardiac chamber prostheses according to the invention has been implanted; and
Figure 12 shows a schematic sectional view of a heart in which a fourth embodiment of the assembly of cardiac chamber prostheses according to the invention has been implanted.

In the Figures identical reference numbers will be used for alike elements.

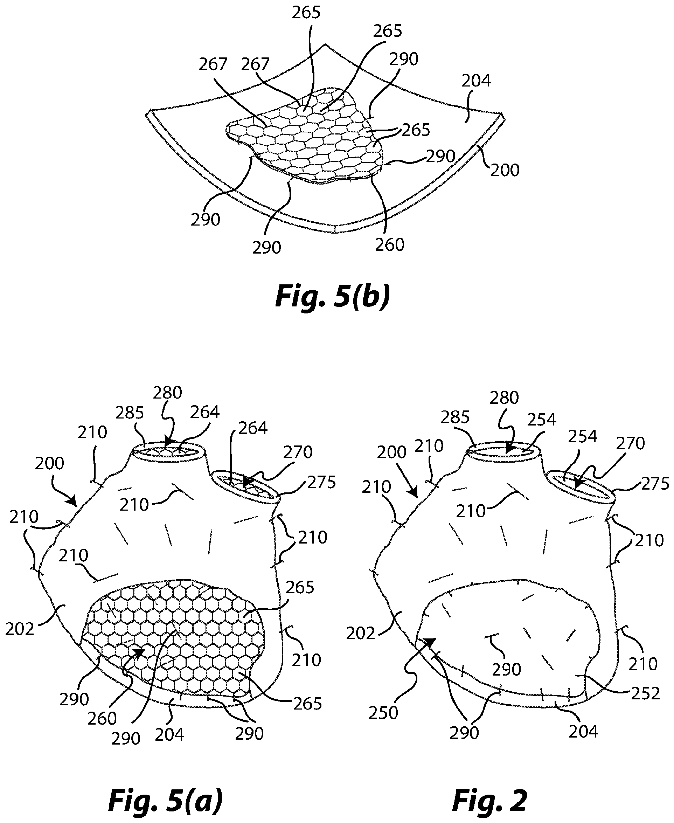

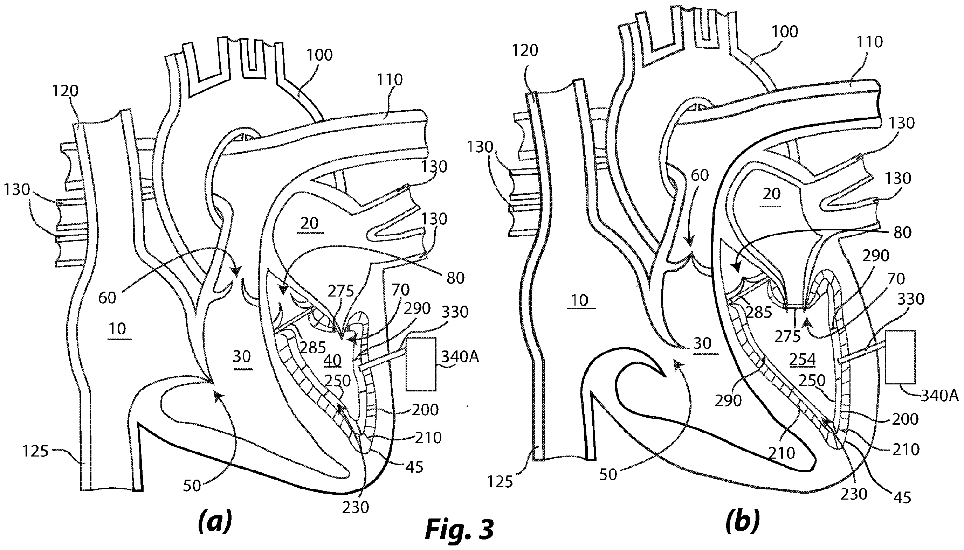

With reference to Figures 2 and 3, a first embodiment of the prosthesis according to the invention can be observed, that is intended for the left ventricle, thereby it is a left ventricle prosthesis. The left ventricle prosthesis comprises an outer elastic membrane 200 and an inner elastic membrane 250, each provided with a first aperture and a second aperture. The borders of the first apertures of the outer and inner elastic membranes 200 and 250 are integrally coupled to each other to form an aortic border 285 delimiting an aortic aperture 280; the borders of the second apertures of the outer and inner elastic membranes 200 and 250 are integrally coupled to each other to form a mitral border 275 delimiting a mitral aperture 270. Advantageously, the borders of the first apertures of the outer and inner elastic membranes 200 and 250 are electro-welded to each other to form the aortic border 285; similarly, the borders of the second apertures of the outer and inner elastic membranes 200 and 250 are electro-welded to each other to form the mitral border 275.

The outer elastic membrane 200 and the inner elastic membrane 250 are elastically connected to each other by means of a plurality of elastic ties 290, each having two ends integrally coupled to the inner surface 204 of the outer elastic membrane 200 and to the outer surface 252 of the inner elastic membrane 250, respectively. Each of the elastic ties 290 is configured to be elastically elongated, when subjected to a stress that causes its ends to move away from each other, starting from a rest configuration. The outer surface 202 of the outer elastic membrane 200 is provided with a plurality of conventional hook fasteners 210 (also known as clips), similar to those used to secure the left ventricular volume reduction prostheses to the inner wall 45 of the left ventricle, which are configured to grip the inner wall 45 of the left ventricle by sticking into the tissue of the left ventricle, and allowing the outer elastic membrane 200 to be stably implanted there, as shown in Figure 3.

Both the outer elastic membrane 200 and the inner elastic membrane 250 have each a morphology that approximates the one of the inner wall 45 of a left ventricle (in which the prosthesis is intended to be implanted), whereby they substantially comprise three areas: a base where the first and second apertures are arranged, a ventricular apex and an intermediate wall that joins the base and the ventricular apex, thereby the intermediate wall is arranged in an intermediate position between the base and the ventricular apex. This allows the outer elastic membrane 200 to fit like an inner glove to the inner wall of the ventricle to which it is configured to grip through the clips 210.

In this regard, the left ventricle prosthesis, namely the outer elastic membrane 200 and its inner elastic membrane 250, can be made in various sizes to adapt to the specific volumes of the left ventricle of different patients, which are linked to the patient's body surface (BSA - Body Surface Area).

Thanks to such shape of the outer and inner elastic membranes 200 and 250, the prosthesis is configured to be implanted inside the left ventricle by adapting to its tissue, to the inner wall 45 of which it is configured to grip through the clips 210. Optionally, the shape of the lateral surface of the two outer and inner elastic membranes 200 and 250 can include two notches (not shown in the Figures), without clips, configured to receive the anterior and posterior papillary muscles and the tendinous cords of the left ventricle (also not shown in the Figures ) connected to the mitral valve 70 in order not to make the prosthesis interfere with the papillary muscles and tendinous cords themselves.

In any case, the two outer and inner elastic membranes 200 and 250 must not necessarily have a morphology exactly conforming to the one of the inner wall 45 of a left ventricle (in which the prosthesis is intended to be implanted), since their elasticity allows to compensate any morphological differences; in particular, the elasticity of the outer elastic membrane 200 allows it to follow the morphology of the inner wall 45 of the left ventricle which it grip through the clips 210.

The mitral border 275 is configured to surround and be sutured on the native mitral valve 70 and the aortic border 285 is configured to surround and be sutured on the native aortic valve 80. In particular, the mitral border 275 operates as inlet border, the native mitral valve 70 operates as native inlet valve of which the at least one inlet opening of the cardiac chamber consists, the aortic border 285 operates as outlet border, and the native aortic valve 80 operates as native outlet valve.

The outer and inner elastic membranes 200 and 250 and the ties 290 are made of biocompatible elastic materials, optionally biocompatible elastic polymeric materials, more optionally polytetrafluoroethylene (also known as ePTFE).

The clips 210 are made of biocompatible metal material, optionally a shape memory alloy, more optionally a nickel titanium shape memory alloy (also known as NITINOL).

The outer and inner elastic membranes 200 and 250, elastically connected to each other by means of the plurality of elastic ties 290, delimit a closed interspace 230 between them having a volume configured to be elastically variable, as it will be described in detail later. In particular, the reference support elastic membrane structure consists of the outer elastic membrane 200, the interspace 230 operates as primary interspace and the plurality of elastic ties 290 operates as plurality of primary variable connection elements.

Advantageously, the outer elastic membrane 200 has a coefficient of elasticity not greater (i.e. lower than or equal to), optionally lower (i.e. it is less elastic), than the coefficient of elasticity of the inner elastic membrane 250. Also, in order to better simulate the physiological elastic behaviour of the left ventricle, the coefficient of elasticity of the inner elastic membrane 250 is optionally variable according to the area thereof, as it will be described in greater detail later.

Advantageously, the elastic ties 290 have a coefficient of elasticity greater than the one of the outer and inner elastic membranes 200 and 250. Still in order to better simulate the physiological elastic behaviour of the left ventricle, the coefficient of elasticity of the elastic ties 290 is optionally variable according to the area of the inner elastic membrane 250 to which they are connected with one end thereof, as it will be described in greater detail later.

The left ventricle prosthesis shown in Figures 2 and 3 can be implanted in the left ventricle by means of a minimally invasive percutaneous implant, by inserting it through the aortic valve 80 by means of a catheter, optionally having a diameter ranging from 18 to 24 French, more optionally ranging from 18 to 21 French (where French is the unit of measurement of catheter diameters according to the so-called French scale, also known as the Charrière scale, where 1 French = 0,33 mm), that will internally contain the compacted prosthesis. This insertion is similar to those conventionally currently made for the insertion of other devices, such as artificial aortic valves, mitral clips, and peripheral stents.

After insertion into the left ventricle, the prosthesis is expanded and the clips 210 with which the outer surface 202 of the outer elastic membrane 200 is provided grip the inner wall 45 of the left ventricle so as to stably position the prosthesis in the left ventricle itself. The mitral border 275, that is positioned so as to surround the mitral valve 70, is sutured in proximity of the mitral valve 70 itself; similarly, the aortic border 285, that is positioned so as to surround the aortic valve 80, is sutured in proximity of the aortic valve 80 itself.

Alternatively, the left ventricular prosthesis shown in Figures 2 and 3 can be implanted in the left ventricle by means of non-highly invasive cardiac surgery (e.g. transapical or trans-subclavian one) or by means of invasive cardiac surgery. In this case, following the positioning of the prosthesis by inserting clips 210 into the left ventricle tissue, the prosthesis can also be stabilized by the surgeon by suturing to the inner wall 45 of the left ventricle. Also in this case, the mitral border 275 and the aortic border 285 are sutured in proximity of the mitral valve 70 and the aortic valve 80, respectively.

Advantageously, the left ventricle prosthesis according to the invention comprises portions, e.g. in the outer elastic membrane 200 and/or the inner elastic membrane 250, which are radiopaque, thereby they are visible by radioscopy, to assist the surgeon during the placement of the prosthesis in the left ventricle (for instance, the mitral border 275 and the aortic border 285 can be radiopaque).

The interspace 230 delimited by the outer elastic membrane 200 and the inner elastic membrane 250 is configured to receive a fluid, in particular a liquid, such as a haemocompatible solution, for instance sterile saline solution, or a gas, for instance helium, with varying amount and/or pressure to dynamically modify the volume of the interspace 230 itself and, thus, to modify the elastically variable volume delimited by the inner surface 254 of the inner elastic membrane 250.

To this end, the interspace 230 is accessible through (at least) one access hole arranged on the outer elastic membrane 200, on which hole, after implantation of the prosthesis in the left ventricle, a duct 330 is sutured, wherein the duct 330 connects the interspace 230 to an external (hydraulic or pneumatic) pump 340A. Optionally, the borders of this (at least one) access hole are made of radiopaque material, to allow their easy identification for positioning the duct 330; during insertion of the prosthesis into the left ventricle, this (at least one) access hole is closed to prevent biological material from entering the interspace 230. Said (at least one) hole can be arranged in any position of the outer elastic membrane 200 and the duct 330 can consequently cross a corresponding area of the myocardium (possibly also passing through the aorta 100). Advantageously, the prosthesis can be provided with a plurality of sealed access holes (or simply with borders delimiting an area of the outer elastic membrane 200 that can be removed to make an access hole, on which borders the duct 330 can be sutured), and the plurality of sealed access holes can be distributed on the outer elastic membrane 200: the surgeon can select the access hole on which the duct 330 is to be sutured on the basis of the position on the outer elastic membrane 200 which corresponds to an adequate area of the myocardium, and can open the selected access hole (or remove the area of the outer elastic membrane 200 delimited by the selected border to make the access hole), then suturing the duct 330 on its border. Alternatively, the surgeon can make the hole access in any position on the outer elastic membrane 200 (that corresponds to an adequate area of the myocardium) by removing an area of the outer elastic membrane 200 and then suturing the duct 330 on the border of the hole thus created.

To better understand the invention, the main operating modes of the left ventricle prosthesis shown in Figures 2 and 3 are described below. At the end of an atrial systole, the inner elastic membrane 250 is expanded to the ventricular telediastolic volume and contains oxygenated blood coming from the left atrium 20. Referring to Figure 3a, it can be observed that, in order to produce an efficient ventricular systole, the pump 340A injects fluid into the interspace 230, for instance by introducing liquid (e.g. haemocompatible solution) or by blowing gas (e.g. helium). This causes the volume of the interspace 230 to increase and the inner elastic membrane 250 to move away from the outer elastic membrane 200, i.e. the inner elastic membrane 250 moves towards the inside of the left ventricle. This produces a consequent increase in pressure in the inner elastic membrane 250 (i.e. an increase in the pressure of the blood contained in the inner elastic membrane 250) and, hence, the closing of the mitral valve 70, the opening of the aortic valve 80 and the ejection of the systolic stroke, also known as SV (stroke volume), through the aortic opening 280. In particular, the overall variation in internal volume delimited by the inner elastic membrane 250 ranges, for instance, from about 80 millilitres to about 120 millilitres for a normotype adult, that is equal to the volume of the ejected SV; advantageously, the pump 340A is configured to displace a volume of about 200 millilitres of liquid or gas.

Making reference to Figure 3b, it can be observed that at the end of the ventricular systole the aortic valve 80 closes and the atrial systole begins, wherein the mitral valve 70 opens and the inside of the inner elastic membrane 250 begins to fill with blood coming from the left atrium 20 that passes through the mitral aperture 270. In order to produce an efficient ventricular diastole, the pump 340A removes fluid from the interspace 230, for instance by aspirating liquid (e.g. haemocompatible solution) or gas (e.g. helium). This causes the volume of the interspace 230 to decrease and the inner elastic membrane 250 to get close to the outer elastic membrane 200, i.e. the inner elastic membrane 250 moves towards the inner wall 45 of the left ventricle. This maintains the pressure in the inner elastic membrane 250 (i.e. the pressure of the blood contained in the inner elastic membrane 250) at lower values than the blood pressure in the aorta 100 and the blood pressure in the left atrium 20, keeping the aortic valve 80 closed and the mitral valve 70 open and promoting the blood flow from the left atrium 20 inside the inner elastic membrane 250. Advantageously, the inner elastic membrane 250 can substantially come into contact with the outer elastic membrane 200 and the inner and outer elastic membranes 250 and 200 can continue to expand with an increase in the overall volume of the left ventricle until an adequate telediastolic volume is reached; alternatively, for instance in case of a myocardium with not dramatically compromised ventricular dilation, the inner elastic membrane 250 can expand reaching the ventricular telediastolic volume without also causing the expansion of the outer elastic membrane 200 as well.

At the end of the atrial systole, the blood stops flowing from the left atrium 20 inside the inner elastic membrane 250, the mitral valve 70 closes and the aortic valve 80 opens, thus starting a new cardiac cycle.

The prosthesis allows to obtain an efficient emptying of the inside of the inner elastic membrane 250 during ventricular systole, thanks to the introduction of fluid into the interspace 230, and a better emptying of the left atrium 20 and an efficient filling of the inside of the inner elastic membrane 250 during atrial systole, thanks to the removal of fluid from the interspace 230.

In other words, the outer elastic membrane 200, integral with the inner wall 45 of the left ventricle, operates as a pivot for the inner elastic membrane 250 assisting its displacements towards the centre of the left ventricle and in the opposite direction towards the inner wall 45 of the left ventricle, allowing a reliable, rapid and efficient variation of the volume of the interspace 230. For this reason, the outer elastic membrane 200 advantageously has a lower coefficient of elasticity than the one of the inner elastic membrane 250, so as to be more rigid than the latter and have limited expansions.

As stated, in order to better simulate the physiological elastic behaviour of the left ventricle, the coefficient of elasticity of the inner elastic membrane 250 is optionally variable depending on the area. In fact, the ventricular apex has greater displacements than the intermediate areas and, even more, with respect to the areas of the mitral valve 70 and the aortic valve 80, which are the most rigid since the flow lines from the first to the second are not long. Consequently, optionally the base of the inner elastic membrane 250 has a lower coefficient of elasticity than the one of the intermediate wall of the inner elastic membrane 250, and the coefficient of elasticity of the intermediate wall of the inner elastic membrane 250 is lower than the one of the ventricular apex of the inner elastic membrane 250 (that has the maximum coefficient of elasticity). This permits to avoid creating potential flow turbulence and energy losses. Possibly, the elastic coefficient of the inner elastic membrane 250 can also vary in a continuous way (gradually increasing) going from the base to the ventricular apex of the inner elastic membrane 250.

Still in order to better simulate the physiological elastic behaviour of the left ventricle, the coefficient of elasticity of the elastic ties 290 is optionally variable according to the area of the inner elastic membrane 250 to which they are connected with one of their ends, namely: the elastic ties 290 connected to the base of the inner elastic membrane 250 have a coefficient of elasticity lower than the one of the elastic ties 290 connected to the intermediate wall of the inner elastic membrane 250, and the coefficient of elasticity of the elastic ties 290 connected to the intermediate wall of the inner elastic membrane 250 is lower than the one of the elastic ties 290 connected to the ventricular apex of the inner elastic membrane 250 (which have the maximum coefficient of elasticity). Possibly, the elastic coefficient of the elastic ties 290 can also vary in a continuous way (progressively increasing) going from the base to the ventricular apex of the inner elastic membrane 250.

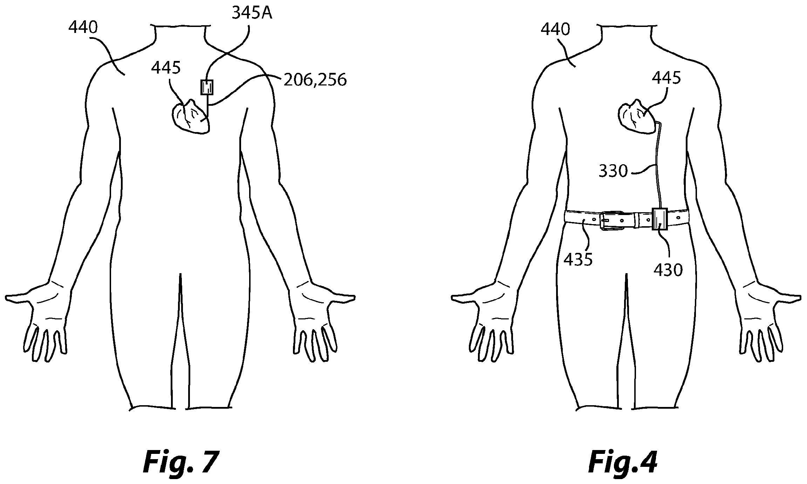

As shown in Figure 4, the pump 340A is housed, together with the, possibly rechargeable, batteries or accumulators dedicated to its power supply, in a box (or even a backpack) 430 carried by the patient 440, for instance attached to a belt 435. The duct 330 connects the pump 340A housed in the box 430 with the left ventricle prosthesis implanted in the heart 445 of the patient 440. The pump 340A is provided with an electronic processing unit that controls the operation of the pump itself so as to regulate the expansion and compression of the inner elastic membrane 250 with amplitudes and speeds adaptable to the specific BSA of the patient 440 in which the prosthesis according to the invention is implanted. In this regard, the operation of the pump 340A, and, consequently, the volume changes of the interspace 230, can be controlled in a similar way to what happens in a pacemaker.

The cardiac assistance system comprising the left ventricle prosthesis and the pump 340A (controlled by the electronic processing unit) can be used as a semi-permanent bridge, i.e. as a transient support system until a new cardiac organ is available for transplantation, or as a permanent prosthetic system as an alternative to the replacement of the entire cardiac organ.

A significant advantage offered by the prosthesis according to the invention over the prior art solutions is that the patient continues to have the native cardiac organ, despite its reduced and conditioned functionality. Also thanks to the less invasive nature of the prosthesis according to the invention, this allows to maintain or restore a correct functionality of the other cardiac chambers which could begin to have dysfunctions caused by left ventricular dysfunction. By way of example, a left ventricular dysfunction over time can lead to degeneration and hence also to a pathology of the left atrium; even correcting the left ventricle alone, it is possible to reestablish an adequate work of the left atrium, as well as of the right ventricle and, consequently, of the right atrium. In this way, the prosthesis according to the invention allows to recover the rest of the organ that is not yet totally compromised.

Figure 5 shows a second embodiment of the prosthesis according to the invention that is also intended for the left ventricle, thereby it is a left ventricle prosthesis, that differs from the first embodiment shown in Figures 2 and 3 in the conformation of the inner elastic membrane. In particular, while the inner elastic membrane 250 of the first embodiment has a continuous structure, the inner elastic membrane 260 of the second embodiment shown in Figure 5 has a honeycomb discontinuous structure formed by a plurality of elastic plates 265, optionally hexagonal ones, connected to each other along elastic junction lines 267.

Other embodiments of the prosthesis according to the invention can have the inner elastic membrane that has a discontinuous structure different from that shown in Figure 5, for instance formed by a plurality of elastic polygonal plates, such as pentagons and/or hexagons, connected to each other along elastic junction lines.

Advantageously, the elastic ties 290 connecting the inner surface 204 of the outer elastic membrane 200 to the outer surface of the inner elastic membrane 260 are fixed to apices (i.e. vertices) of elastic plates 265 (coinciding with ends of elastic junction lines 267) forming the honeycomb discontinuous structure (or, more generally, to apices of elastic polygonal plates forming the discontinuous structure).

Optionally, the coefficient of elasticity of the elastic junction lines 267 is greater than the one of the elastic plates 265; in this case, the minimum volume delimited by the inner elastic membrane 260 is always greater than zero, increasing the reliability and efficiency of the operation of the prosthesis.

Further embodiments of the prosthesis according to the invention may have the outer elastic membrane which has a discontinuous structure formed by a plurality of elastic plates connected to each other, wherein such discontinuous structure can have a honeycomb conformation, e.g. with hexagonal plates, or a polygonal plates one, such as with pentagons and/or hexagons. Advantageously, the elastic ties connecting the inner surface of the outer elastic membrane to the outer surface of the inner elastic membrane are fixed to apices of elastic plates (coinciding with ends of elastic junction lines) forming the discontinuous structure of the outer elastic membrane. Also, the outer elastic membrane can be possibly perforated. The embodiments with an outer elastic membrane having a discontinuous structure can be provided with an inner elastic membrane having either a continuous or a discontinuous structure.

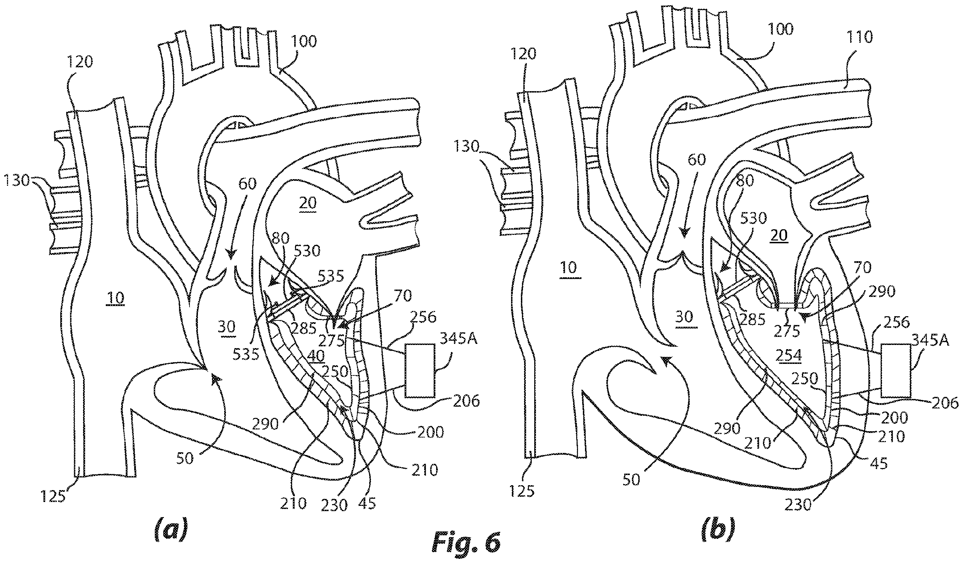

With reference to Figure 6, a third embodiment of the prosthesis according to the invention can be observed, still intended for the left ventricle, thereby it is a left ventricle prosthesis. Instead of a hydraulic or pneumatic control of the volume delimited by the interspace 230 (through a pump 340A which introduces or sucks a fluid into or from it), such third embodiment of the prosthesis according to the invention has the outer elastic membrane 205 and the inner elastic membrane 255 each provided with a plurality of, optionally electrically insulated, electrodes, configured to electrically and/or electromagnetically polarise the outer and inner elastic membranes 205 and 255 themselves, under control of an electronic processing unit 345A connected to the electrodes of the outer and inner elastic membranes 205 and 255 through respective control cables 206 and 256.

The variation in the volume of the interspace 230 is controlled by the electrical and/or electromagnetic polarity of the outer and inner elastic membranes 205 and 255. During the ventricular systole (shown in Fig. 6a), the electronic processing unit 345A controls the electrodes so that the outer and inner elastic membranes 205 and 255 have the same electrical and/or electromagnetic polarity, thereby they reject each other and increase the volume of the interspace 230, causing the ejection of the systolic stroke into the aorta 100 similarly to what described with reference to Figure 3a. During the atrial systole (shown in Fig. 6b), the electronic processing unit 345A controls the electrodes so that the outer and inner elastic membranes 205 and 255 have opposite electrical and/or electromagnetic polarity, thereby they attract each other and reduce the volume of the interspace 230 (possibly until zeroing), promoting the blood flow from the left atrium 20 inside the inner elastic membrane 255 similarly to what described with reference to Fig. 3b.

In this regard, the interspace 230 can be filled with a liquid (e.g. haemocompatible solution) and is in fluid communication with at least one variable volume elastic bag (not shown in the Figures), operating as a liquid reservoir, that can be positioned inside the myocardium (for instance in proximity to the aortic valve 80 and/or in proximity to the mitral valve 70) or outside the myocardium; during atrial systole, the liquid is displaced from the interspace 230 into the elastic bag (which increases its volume), and during ventricular systole the liquid is displaced from the elastic bag (which decreases its volume) into the interspace 230. Alternatively to, or in combination with, the liquid, the interspace 230 can be filled with a gas (e.g. helium) that, since it is compressible, can vary its pressure from a high value, during the atrial systole, to a low value, during the ventricular systole, without necessarily requiring that the interspace 230 be in fluid communication with a variable volume elastic bag (that, in this case, is thus optional).

The electronic processing unit 345A can control the electric and/or electromagnetic polarity of each one of the outer and inner elastic membranes 205 and 255 in a way that varies in time and in amplitude in function of the specific area of the (outer or inner) elastic membrane (205 or 255) under consideration. In particular, the electronic processing unit 345A can control the electrodes of the outer elastic membrane 205 with the same control signal (also variable over time), or by considering the electrodes subdivided into distinct groups (for instance, a base group comprising one or more electrodes arranged in the base of the outer elastic membrane 205, an intermediate wall group comprising one or more electrodes arranged in the intermediate wall of the outer elastic membrane 205, and a ventricular apex group comprising one or more electrodes arranged in the ventricular apex of the outer elastic membrane 205) each of which is controlled by a respective control signal (also variable over time), or by sending a corresponding control signal (also variable over time) to each one of the electrodes (wherein possibly two or more electrodes can receive corresponding control signals identical to each other). Similarly, the electronic processing unit 345A can also control the electrodes of the inner elastic membrane 255 in similar modes, i.e. with the same control signal (also variable over time), or with respective control signals (also variable over time) for distinct groups into which the electrodes are subdivided, or with corresponding control signals (also variable over time) for each one of the electrodes. In this regard, the electrode control mode of the outer elastic membrane 205 can be the same as or different from the electrode control mode of the inner elastic membrane 255.

The power supply of the electronic processing unit 345A that controls the operation of the prosthesis occurs through, optionally rechargeable, batteries (or accumulators) and can be housed in an external box (or even a backpack) carried by a patient (in a similar way to what shown in Figure 4). Alternatively, as shown in Figure 7, the electronic processing unit 345A, along with the batteries for its power supply, can be subcutaneously implanted in a patient 440 in whose heart 445 the left ventricular prosthesis is implanted, similarly to a conventional pacemaker. In this regard, the electrodes and, consequently, the polarities, of the outer and inner elastic membranes 205 and 255 and the resulting volume variations of the interspace 230 can be controlled similarly to what happens in a pacemaker. Also in this case, the electronic processing unit 345A controls the operation of the prosthesis so as to regulate the expansion and compression of the inner elastic membrane 255 with amplitudes and speeds adaptable to the specific BSA of the patient 440 in which the prosthesis according to the invention is implanted, for instance by adjusting the intensities of electric and/or electromagnetic polarity to cause a variable attraction and repulsion between the outer and inner elastic membranes 205 and 255.

The cardiac assistance system including the left ventricle prosthesis of Figure 6 and the electronic processing unit 345A controlling its operation can also be used as a semi-permanent bridge or as a permanent prosthetic system.

The left ventricle prosthesis shown in Figure 6 is further provided with an artificial aortic valve 530 integrally coupled to the aortic border 285 delimiting the aortic aperture 280 of the prosthesis. Such artificial aortic valve 530 allows to overcome the problems caused by an inadequate or pathological native aortic valve. In this case, the prosthesis is advantageously implanted with a cardiac surgery technique, by opening the native aortic valve, or, alternatively, by a minimally invasive percutaneous route or by a non-highly invasive transapical or trans-subclavian route. Once implanted, the artificial aortic valve 530 pushes the natural one maintaining it open, thereby the opening or closing of the aortic aperture 280 of the prosthesis depends on the opening (as shown in Fig.6a, where the opening of the artificial aortic valve 530 is schematically represented by raised slabs 535) or the closure (as shown in Fig.6b) of the artificial aortic valve 530.

In particular, the artificial aortic valve 530 integrally coupled to the aortic border 285 delimiting the aortic aperture 280 of the prosthesis according to the invention has a better positioning and greater stability than what obtainable with the prior art artificial aortic valves. In particular, the operation of the artificial aortic valve 530 could also be controlled by specific electrodes distributed on the components of the valve itself which are configured to cause the valve to open and close through an electrical and/or electromagnetic polarisation of such components, wherein the electrodes are in turn controlled by the electronic processing unit 345A. Also, the artificial aortic valve 530 avoids the problems of residual regurgitation after implantation which can occur with a conventional percutaneous artificial aortic valve implant, also known as TAVI (Trans Aortic Valve Implantation).

Alternatively or in combination with the artificial aortic valve 530, other embodiments of the prosthesis according to the invention can be provided with an artificial mitral valve. Also the embodiments of the prosthesis according to the invention in which the volume delimited by the interspace 230 is controlled by a pump that introduces or sucks a fluid into or from it can be provided with artificial valves.

It must be noted that other embodiments of the prosthesis according to the invention can use both a hydraulic or pneumatic control, as shown in Figures 3 and 4, and an electrical and/or electromagnetic control, as shown in Figures 6 and 7, wherein the two types of controls cooperate in varying the volume delimited by the interspace 230; in particular, in the event that the hydraulic control uses a liquid (e.g. haemocompatible solution), the pump 340A can advantageously replace said at least one variable volume elastic bag (positioned inside or outside the myocardium) of the electrical and/or electromagnetic control. Also, it is possible to have a single electronic processing unit controlling both the pump 340A and the electrodes of the outer and inner elastic membranes 205 and 255.

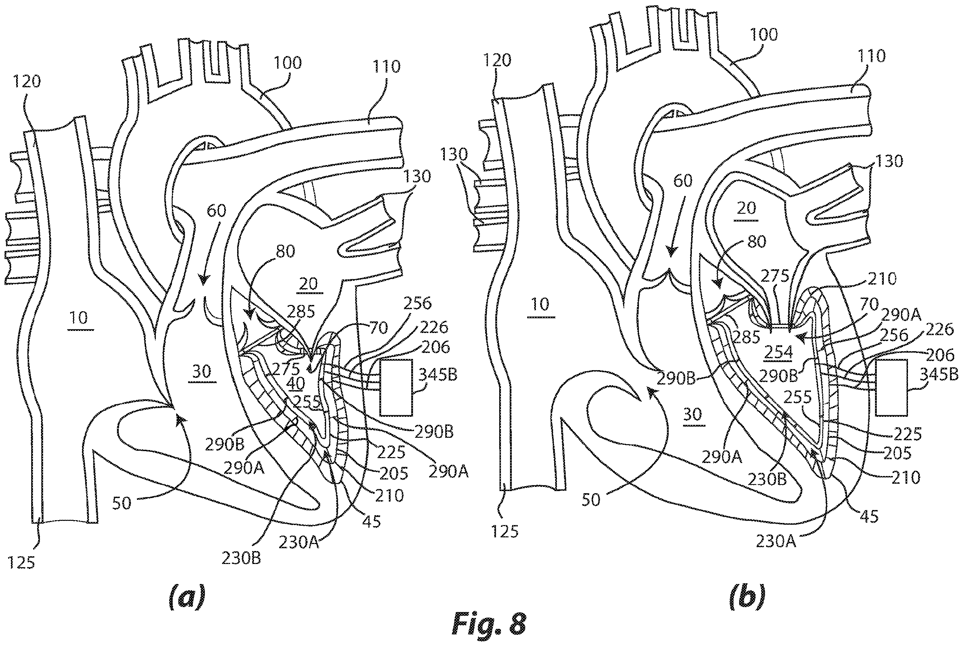

With reference to Figure 8, it is possible to observe a fourth embodiment of the prosthesis according to the invention still intended for the left ventricle, thereby it is a left ventricle prosthesis, that is intended for patients whose myocardium has pathological characteristics that do not allow to use the inner wall 45 of the left ventricle in a sufficiently effective and reliable way, whereby the outer elastic membrane 200, integral with the inner wall 45 of the left ventricle, is not capable to operate sufficiently effectively and reliably as a pivot for the inner elastic membrane 250. By way of example, and not by way of limitation, this is the case of patients with dilated cardiomyopathy.

The left ventricle prosthesis shown in Figure 8 differs from the prosthesis shown in Figures 6-7 in that it further comprises an intermediate elastic membrane 225 that is interposed between the outer elastic membrane 205 and the inner elastic membrane 255. The intermediate elastic membrane 225 has a shape similar to those of the outer elastic membrane 205 and of the inner elastic membrane 255, whereby it is provided with a first aperture and a second aperture the borders of which are integrally coupled to those of the first apertures and the second apertures, respectively, of the outer and inner elastic membranes 205 and 255, to form an aortic border (configured to surround and be sutured on the native aortic valve 80) delimiting an aortic aperture of the left ventricle prosthesis and a mitral border (configured to surround and be sutured on the native mitral valve 70) delimiting a mitral aperture of the left ventricle prosthesis. Also in this case, advantageously the borders of the first apertures of the outer, intermediate and inner elastic membranes 205, 225 and 255 are electro-welded to each other to form the aortic border; similarly, the borders of the second apertures of the outer, intermediate and inner elastic membranes 205, 225 and 255 are electro-welded to each other to form the mitral border.

Similarly to the outer and inner elastic membranes 205 and 255, even the intermediate elastic membrane 225 is provided with a plurality of, optionally electrically insulated, electrodes configured to electrically and/or electromagnetically polarise the intermediate elastic membrane 225 itself, under control of an electronic processing unit 345B, that also controls the electrical and/or electromagnetic polarisation of the outer and inner elastic membranes 205 and 255 and that is connected to the electrodes of the outer, intermediate and inner elastic membranes 205, 225 and 255 by means of respective control cables 206, 226 and 256.

The intermediate elastic membrane 225 is elastically connected to the outer elastic membrane 205 and to the inner elastic membrane 255 by means of a plurality of outer elastic ties 290A and a plurality of inner elastic ties 290B, respectively. Each one of the outer elastic ties 290A has two ends integrally coupled to the inner surface of the outer elastic membrane 205 and to the outer surface (i.e. the surface facing the outer elastic membrane 205) of the intermediate elastic membrane 225, respectively; similarly, each one of the inner elastic ties 290B has two ends integrally coupled to the outer surface of the inner elastic membrane 255 and to the inner surface (i.e. the surface facing the inner elastic membrane 205) of the intermediate elastic membrane 225, respectively. Each one of the inner elastic ties 290B is configured to be elastically elongated, when subjected to a stress that causes its ends to move away from each other, starting from a rest configuration; advantageously, also each one of the outer elastic ties 290A is configured to be elastically elongated, when subjected to a stress that causes its ends to move away from each other, starting from a rest configuration. Optionally, the coefficient of elasticity of the outer elastic ties 290A is greater than the coefficient of elasticity of the intermediate elastic membrane 225 and the coefficient of elasticity of the outer elastic membrane 205; more optionally, the coefficient of elasticity of the outer elastic ties 290A varies in function of the area of the intermediate elastic membrane 225 to which they are connected.

As stated, the outer and inner elastic membranes 205 and 255 are like those of the embodiment shown in Figures 6-7, whereby the outer surface of the outer elastic membrane 205 is provided with a plurality of conventional clips 210 which are configured to grip to the inner wall 45 of the left ventricle, by sticking into the tissue of the left ventricle, and allowing the outer elastic membrane 205 to be stably implanted.

Also the intermediate elastic membrane 225, as the outer and inner elastic membranes 205 and 255, advantageously has a morphology which approximates the one of the inner wall 45 of a left ventricle (in which the prosthesis is intended to be implanted), optionally comprising two notches configured to receive the anterior and posterior papillary muscles and the tendinous cords of the left ventricle connected to the mitral valve 70, and can be made in various sizes to adapt to the specific volumes of the left ventricle of different patients, which are related to the patient's BSA. In any case, similarly to the two outer and inner elastic membranes 205 and 255, even the intermediate elastic membrane 225 must not necessarily have a morphology exactly conforming to the one of the inner wall 45 of a left ventricle (in which the prosthesis is intended to be implanted), since its elasticity allows to compensate for any morphological differences.

Moreover, the intermediate elastic membrane 225, as well as the outer and inner elastic membranes 205 and 255, as well as the outer elastic ties 290A and the inner elastic ties 290B are made of biocompatible elastic materials, optionally biocompatible elastic polymeric materials, more optionally polytetrafluoroethylene (also known such as ePTFE).

The outer and intermediate elastic membranes 205 and 225, elastically connected to each other by means of the plurality of outer elastic ties 290A, delimit a closed outer interspace 230A between them having a volume configured to be elastically variable (even if this is not an essential feature for the invention). The intermediate and inner elastic membranes 225 and 255, elastically connected to each other by means of the plurality of inner elastic ties 290B, delimit a closed inner interspace 230B between them having a volume configured to be elastically variable, as t will be described in detail later.

In particular, the reference support elastic membrane structure comprises the outer elastic membrane 205, the intermediate elastic membrane 225 and the plurality of outer elastic ties 290A. Also, the inner interspace 230B operates as a primary interspace, the outer interspace 230A operates as a secondary interspace, the plurality of inner elastic ties 290B operates as a plurality of primary variable connection elements, and the plurality of outer elastic ties 290A operates as a plurality of secondary variable connection elements.

The intermediate elastic membrane 225 can have both a continuous structure and a discontinuous structure formed by a plurality of elastic plates connected to each other, wherein such discontinuous structure can have a honeycomb conformation, e.g. with hexagonal plates, or a polygonal plate one, such as with pentagons and/or hexagons. Advantageously, the elastic ties connecting the inner surface of the outer elastic membrane to the outer surface of the intermediate elastic membrane and the elastic ties connecting the outer surface of the inner elastic membrane to the inner surface of the intermediate elastic membrane are fixed to apices of elastic plates (coinciding with ends of elastic junction lines) forming the discontinuous structure of the intermediate elastic membrane. The embodiments with an intermediate elastic membrane having a discontinuous structure can be provided with an inner elastic membrane having either a continuous or a discontinuous structure and an outer elastic membrane having either a continuous or a discontinuous structure.

Also in the fourth embodiment of the prosthesis shown in Figure 8, similarly to the one shown in Figure 6, the electronic processing unit 345B controls the electrical and/or electromagnetic polarisation of all and each of the outer, intermediate and inner elastic membranes 205, 225 and 255 so as to vary the volume of the inner interspace 230B. In particular, the intermediate elastic membrane 225 has coefficient of elasticity not greater (i.e. lower than or equal to), optionally lower (i.e. it is less elastic), than the coefficient of elasticity of the outer elastic membrane 205. This allows the intermediate elastic membrane 225 to operate as pivot for the inner elastic membrane 255, assisting its displacements towards the centre of the left ventricle and in the opposite direction towards the inner wall 45 of the left ventricle, allowing a reliable, rapid and efficient overall variation of the inner volume delimited by the inner elastic membrane 255. In particular, with respect to the configuration (optionally the rest one) that it assumes in the substantially static (i.e. constant) reference position, the intermediate elastic membrane 225 is optionally configured to expand only a little, with respect to the expansion amplitude of the outer and inner elastic membranes 205 and 255, and substantially not to compress at all.

In this regard, the electronic processing unit 345B controls the electrical and/or electromagnetic polarisation of all and each of the outer, intermediate and inner elastic membranes 205, 225 and 255 so that the intermediate elastic membrane 225 assumes a substantially static (i.e. constant) position that internally defines the physiological ventricular telediastolic volume of the patient, and so that the inner elastic membrane 255 moves with respect to the intermediate elastic membrane 225, cyclically varying the volume of the inner interspace 230B. In particular, during the atrial systole (shown in Fig.8b), the electronic processing unit 345B controls the electrical and/or electromagnetic polarisation of all and each of the outer, intermediate and inner elastic membranes 205, 225 and 255 so that the inner and intermediate elastic membranes 255 and 225 attract each other and reduce the volume of the inner interspace 230B (optionally) to zero, promoting the blood flow from the left atrium 20 inside the inner elastic membrane 255 similarly to what described with reference to Fig. 3b. During the ventricular systole (shown in Fig.8a), the electronic processing unit 345B controls the electrical and/or electromagnetic polarisation of all and each of the outer, intermediate and inner elastic membranes 205, 225 and 255 so that the intermediate and inner elastic membranes 225 and 255 reject each other and increase the volume of the inner interspace 230B, causing the ejection of the systolic stroke into the aorta 100 similarly to what described with reference to Figure 3a.

In a first mode of operation, the electronic processing unit 345B keeps the polarisation of the intermediate elastic membrane 225 constant and dynamically varies the polarisation of the outer elastic membrane 205 and of the inner elastic membrane 255. In particular, during a cardiac cycle, the electronic processing unit 345B also dynamically varies the polarisation of the outer elastic membrane 205 to prevent the polarity changes of the inner elastic membrane 255 from causing a displacement of the intermediate elastic membrane 225 with respect to the substantially static (i.e. constant) reference position that internally defines the physiological ventricular telediastolic volume of the patient. In other words, in such first mode of operation, the volume of the outer interspace 230A remains substantially constant.

Other modes of operation of the prosthesis shown in Figure 8 may have the electronic processing unit 345B that also dynamically varies the polarisation of the intermediate elastic membrane 225, instead of keeping it constant. Even in these other modes of operation, the volume of the outer interspace 230A can remain substantially constant.

Furthermore, further modes of operation of the prosthesis shown in Figure 8 can have the electronic processing unit 345B that controls the electrical and/or electromagnetic polarisation of all and each of the outer, intermediate and inner elastic membranes 205, 225 and 255 so as to vary also the volume of the outer interspace 230A, in addition to the volume of the inner interspace 230B.

Similarly to the interspace 230 of the prosthesis of Figure 6, the inner interspace 230B can be filled with a liquid (e.g. haemocompatible solution) and is in fluid communication with at least one variable volume elastic bag (not shown in the Figures), operating as liquid reservoir, that can be positioned inside the myocardium (for instance in proximity to the aortic valve 80 and/or in proximity to the mitral valve 70) or outside the myocardium; during the atrial systole, the liquid is moved from the inner interspace 230B into the elastic bag (that increases its volume), and during the ventricular systole the liquid is moved from the elastic bag (that decreases its volume) into the inner interspace 230B. Alternatively to, or in combination with, the liquid, the inner interspace 230B can be filled with a gas (e.g. helium) which, since it is compressible, can vary its pressure from a high value, during the atrial systole, to a low value, during the ventricular systole, without necessarily requiring that the inner interspace 230B be in fluid communication with a variable volume elastic bag (that, in this case, is thus optional).

In the modes of operation wherein the volume of the outer interspace 230A remains substantially constant, this can be filled with a predetermined amount of liquid (e.g. haemocompatible solution) or gas (e.g. helium).

In the other modes of operation of the prosthesis shown in Figure 8 wherein the volume of the outer interspace 230A also varies, this can be filled with a liquid (e.g. haemocompatible solution) and is in fluid communication with at least one variable volume elastic bag (not shown in the Figures) positioned inside the myocardium (for instance in proximity to the aortic valve 80 and/or in proximity to the mitral valve 70) or outside the myocardium, possibly coinciding with said at least one variable volume elastic bag with which the inner interspace 230B is in fluid communication. Alternatively to, or in combination with, the liquid, the outer interspace 230A can be filled with a gas (e.g. helium) that, since it is compressible, can vary its pressure without necessarily requiring that the outer interspace 230A be in fluid communication with a variable volume elastic bag (that, in this case, is thus optional).

A fifth embodiment of the prosthesis according to the invention still intended for the left ventricle differs from that shown in Figure 8 in that, instead of an electrical and/or electromagnetic control of the volume of the inner interspace 230B (and possibly of the volume of the outer interspace 230A) through the polarisation of the outer, intermediate and inner elastic membranes 205, 225 and 255, the volume of the inner interspace 230B (and possibly the volume of the outer interspace 230A) is varied through a hydraulic or pneumatic control, through a pump (or two pumps) that introduces or sucks a fluid into or from the inner interspace 230B (and possibly into the outer interspace 230A), similarly to what illustrated for the prosthesis of Figures 2-5. In this case, the prosthesis can be provided, already before being implanted, with (at least) one portion of duct for accessing the inner interspace 230B that connects (at least) one corresponding access hole present on the outer elastic membrane 205 with the inner interspace 230B; during the implantation phase, the surgeon sutures on the (optionally radiopaque) border of such access hole a corresponding duct that, along with the duct portion, connects the external pump with the inner interspace 230B of which the pump controls the variations of volume.

It must noted that other embodiments of the prosthesis according to the invention intended for the left ventricle and having the outer, intermediate and inner elastic membranes 205, 225 and 255 can use both a hydraulic or pneumatic control and an electric and/or electromagnetic control, wherein the two types of controls cooperate in the variation of the volume delimited by the inner interspace 230B (and possibly of the volume delimited by the outer interspace 230A).

Further embodiments of the prosthesis according to the invention are intended for one of the other three cardiac chambers, i.e. left atrium 20, right ventricle 30 and right atrium 10, and are similar to what illustrated for the prostheses shown in Figures 2-8; namely:
- the prostheses intended for one between the left ventricle 40 and the right ventricle 30 have a first aperture and a second aperture configured to surround and be sutured on the two native valves of the cardiac chamber for which they are intended; optionally, the shape of the lateral surface of the elastic membranes of the prosthesis can include three notches, without clips, configured to receive the papillary muscles of the right ventricle connected to the tricuspid valve 50 in order not to cause the prosthesis to interfere with the papillary muscles themselves;
- the prostheses intended for the left atrium 20 have a first aperture configured to surround and be sutured on the native mitral valve 70, and at least one second aperture configured to be sutured in correspondence of a mouth of at least one respective pulmonary vein 130;
- the prostheses intended for the right atrium 10 have a first aperture configured to surround and be sutured on the native tricuspid valve 50, and one or more second apertures configured to be sutured in correspondence of the superior vena cava 120 and/or the inferior vena cava 125;
- the prostheses have two or three membranes, elastically connected to each other by means of elastic ties, whereby they comprise an outer elastic membrane and an inner elastic membrane, which delimit a closed interspace between them having a volume configured to be elastically variable, or an intermediate elastic membrane interposed between an outer elastic membrane and an inner elastic membrane which delimit with
the intermediate elastic membrane a closed outer interspace and a closed inner interspace having a volume configured to be elastically variable, and wherein the outer elastic membrane is configured to grip to the inner wall of the cardiac chamber through clips;
- the prostheses have at least one pump configured to perform a hydraulic or pneumatic control and/or an electronic processing unit configured to control the electrical and/or electromagnetic polarisation of the elastic membranes, which are provided with electrodes, to control the volume variation of the variable volume interspaces with the related cardiac cycles and phases;
- the prostheses can be provided with at least one artificial valve, similar to the left ventricle prosthesis shown in Figure 6.

In particular, the coefficient of elasticity of the elastic membranes of the prostheses can be different also in function of the specific cardiac chamber for which each prosthesis is intended.

Similarly to what illustrated for the left ventricle prostheses illustrated in Figures 2-8, also the prostheses according to the invention which are intended for one of the other three cardiac chambers, i.e. left atrium 20, right ventricle 30 and right atrium 10, can be implanted with cardiac surgery technique or, alternatively, by a minimally invasive percutaneous route or by a non-highly invasive transapical or trans-subclavian route, through a specific access for the particular cardiac chamber. By way of example, and not by way of limitation, a prosthesis according to the invention intended for the right ventricle, thereby it is a right ventricle prosthesis, can be implanted with cardiac surgery technique by opening the tricuspid valve or the pulmonary valve.

An assembly of cardiac chamber prostheses according to the invention can also be intended for more than one cardiac chamber.

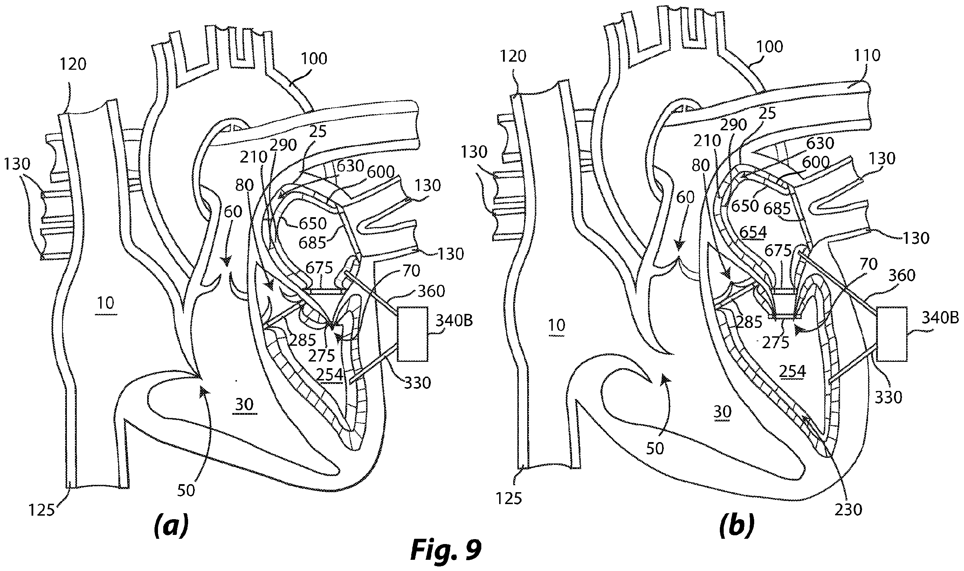

By way of example, Figure 9 shows a first embodiment of the assembly of cardiac chamber prostheses intended for the left ventricle 40 and the left atrium 20. In particular, the assembly comprises a left ventricle prosthesis, similar to that shown in Figure 3, configured to be implanted in a left ventricle 40, and a left atrium prosthesis, also similar to the prosthesis shown in Figure 3, configured to be implanted in a left atrium 20. In particular, the left atrium prosthesis of the assembly shown in Figure 9 comprises an outer elastic membrane 600 and an inner elastic membrane 650, elastically connected to each other by means of a plurality of elastic ties 290, which delimit a closed interspace 630 between them having a volume configured to be elastically variable, and wherein the outer elastic membrane 650 is configured to grip the inner wall 25 of the left atrium 20 through a plurality of clips 210. The outer elastic membrane 600 and the inner elastic membrane 650 of the left atrium prosthesis are provided with a first aperture and a second aperture the borders of which are integrally coupled to each other to form, respectively, a mitral border 675, delimiting a mitral aperture and that is configured to surround and be sutured on the native mitral valve 70, and a border 685 of the pulmonary veins, delimiting an aperture for the outlet mouths of the pulmonary veins 130 and that is configured to surround and be sutured in correspondence of the outlet mouths of the pulmonary veins 130. Other embodiments of the left atrium prosthesis may have two or more second apertures, the borders of which are integrally coupled to each other to form two or more borders of the pulmonary veins delimiting two or more respective apertures for the outlet mouths of the pulmonary veins 130 and each one of which is configured to surround and be sutured in correspondence of an outlet mouth of a respective pulmonary vein 130.

Similarly to what illustrated for the left ventricle prosthesis (shown in Figure 3), the interspace 630 is configured to receive a fluid, namely a liquid, such as a haemocompatible solution, for instance sterile saline solution, or a gas, for instance helium, in a variable amount to dynamically modify the volume of the interspace 630 itself and, hence, to modify the elastically variable volume delimited by the inner surface 654 of the inner elastic membrane 650. To this end, the interspace 630 is accessible through (at least) one access hole arranged on the outer elastic membrane 600, on which hole, after implantation of the prosthesis in the left atrium, a duct 360 is sutured, wherein the duct 360 connects the interspace 630 to an external (hydraulic or pneumatic) pump 340B that is further connected to the interspace 230 of the left ventricle prosthesis through the duct 330; alternatively, the interspace 630 could be connected to an external pump separated from the pump to which the interspace 230 of the left ventricle prosthesis is connected. Optionally, the borders of this (at least one) access hole can be made of radiopaque material and/or the surgeon can make the access hole in any position on the outer elastic membrane 600 similarly to what previously illustrated for the left ventricle prosthesis shown in Figure 3.

In particular, for the left atrium prosthesis of Figure 9, the reference support elastic membrane structure consists of the outer elastic membrane 600, the mitral border 675 operates as outlet border, the native mitral valve 70 operates as native outlet valve, the border 685 of the pulmonary veins operates as inlet border, the outlet mouths of the pulmonary veins 130 operate as inlet aperture of the cardiac chamber, the interspace 630 operates as primary interspace and the plurality of elastic ties 290 operates as plurality of primary variable connection elements.

Other embodiments of the assembly of cardiac chamber prostheses intended for the left ventricle 40 and the left atrium 20 may have, alternatively to or in combination with a hydraulic or pneumatic control through (at least) one pump 340B, an electrical and/or electromagnetic control carried out by an electronic processing unit configured to control the electrical and/or electromagnetic polarisation of the elastic membranes, which are provided with electrodes, of the prostheses of the left ventricle 40 and left atrium 20 similarly to what illustrated for the prosthesis in Figure 6. In this case, the interspaces 230 and 630 can be filled with a liquid (e.g. haemocompatible solution) and can be in fluid communication with each other (instead of or along with at least one variable volume elastic bag), through a communication duct: during the atrial systole, the electrical and/or electromagnetic control increases the volume of the interspace 630 and reduces the volume of the interspace 230, moving the liquid from the latter to the former; during the ventricular systole, the electrical and/or electromagnetic control reduces the volume of the interspace 630 and increases the volume of the interspace 230, moving the liquid from the first to the latter. Optionally, such communication duct between the interspaces 230 and 630 can be sutured by the surgeon during implantation to the prostheses of the left ventricle 40 and left atrium 20 in correspondence of the interspaces 230 and 630, after having filled at least one of these with the liquid.

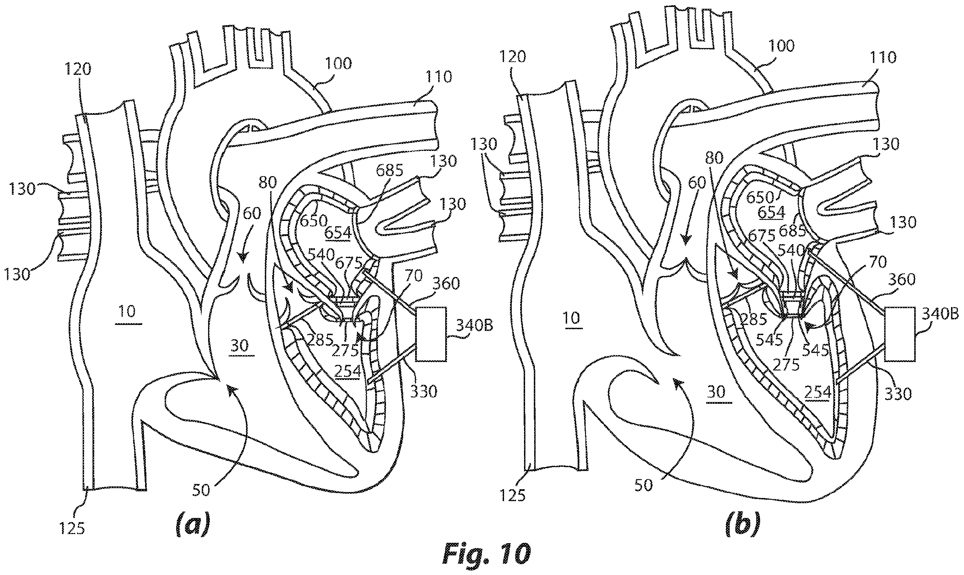

Further embodiments of the assembly of cardiac chamber prostheses may also comprise one or more artificial heart valves. By way of example, and not by way of limitation, Figure 10 shows a second embodiment of the assembly of cardiac chamber prostheses intended for the left ventricle 40 and the left atrium 20 that differs from that shown in Figure 9 in that the left atrium prosthesis is provided with an artificial mitral valve 540 integrally coupled to the mitral border 675 delimiting the mitral aperture of the prosthesis. Such artificial mitral valve 540 allows to overcome the problems caused by an inadequate or pathological native mitral valve, whereby the opening or closing of the mitral aperture of the prosthesis depends on the opening (as shown in Fig. 10b, in which the opening of the artificial mitral valve 540 is schematically represented by raised slabs 545) or by the closure (as shown in Fig. 10a) of the artificial mitral valve 540. It must be noted that the two prostheses intended for the left ventricle 40 and the left atrium 20 could be coupled to each other in correspondence of the respective mitral borders 275 and 675, and that in this case the assembly of cardiac chamber prostheses could also be implanted without suturing the mitral border 275 and/or the mitral border 675 on the native mitral valve 70.

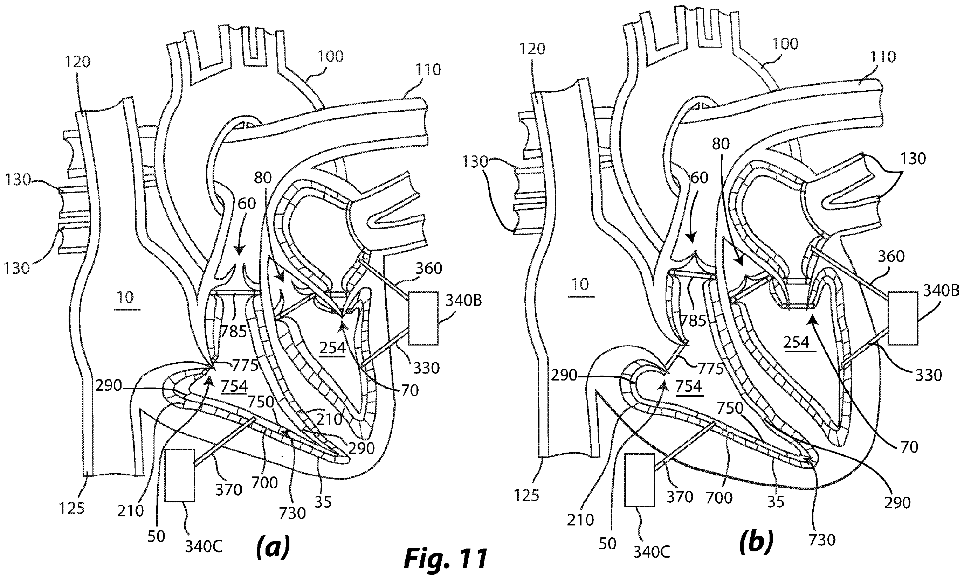

Figure 11 shows a third embodiment of the assembly of cardiac chamber prostheses intended for the left ventricle 40, the right ventricle 30 and the right atrium 10. In particular, the assembly comprises a left ventricle prosthesis and a left atrium prosthesis, similar to those shown in Figure 9, and a right ventricle prosthesis, similar to the prosthesis shown in Figure 3, configured to be implanted in a right ventricle 30. In particular, the right ventricle prosthesis of the assembly shown in Figure 11 includes an outer elastic membrane 700 and an inner elastic membrane 750, elastically connected to each other by means of a plurality of elastic ties 290, which delimit a closed interspace 730 between them having a volume configured to be elastically variable, and wherein the outer elastic membrane 750 is configured to grip the inner wall 35 of the right ventricle 30 through a plurality of clips 210. The outer elastic membrane 700 and the inner elastic membrane 750 of the right ventricle prosthesis are provided with a first aperture and a second aperture the borders of which are integrally coupled to each other to form, respectively, a pulmonary valve border 785, delimiting a pulmonary valve aperture and that is configured to surround and be sutured on the native pulmonary valve 60, and a tricuspid valve border 775, delimiting a tricuspid valve aperture and that is configured to surround and be sutured over the native valve tricuspid 50.

In particular, for the right ventricular prosthesis of Figure 11, the reference support elastic membrane structure consists of the outer elastic membrane 700, the pulmonary valve border 785 operates as outlet border, the native pulmonary valve 60 operates as native outlet valve, the tricuspid valve border 775 operates as inlet border, the tricuspid valve operates as native inlet valve of which the at least one inlet aperture of the cardiac chamber consists, the interspace 730 operates as primary interspace and the plurality of elastic ties 290 operates as plurality of primary variable connection elements.

Similarly to what illustrated for the left ventricle prosthesis (shown in Figure 3), the interspace 730 is configured to receive a fluid, namely a liquid, such as a haemocompatible solution, for instance sterile saline solution, or a gas, for instance helium, in a variable amount to dynamically modify the volume of the interspace 730 itself and, thus, to modify the elastically variable volume delimited by the inner surface 754 of the inner elastic membrane 750. To this end, the interspace 730 is accessible through (at least) one access hole arranged on the outer elastic membrane 700, on which hole, after implantation of the prosthesis in the right atrium, a duct 370 is sutured, wherein the duct 370 connects the interspace 730 to an external (hydraulic or pneumatic) pump 340C. Optionally, the borders of this (at least one) access hole can be made of radiopaque material and/or the surgeon can make the access hole in any position on the outer elastic membrane 700 similarly to what previously illustrated for the left ventricle prosthesis shown in Figure 3.

Also in this case, alternatively to or in combination with the hydraulic or pneumatic control, the prostheses assembly of Figure 11 can have an electrical and/or electromagnetic control carried out by an electronic processing unit configured to control the electrical and/or electromagnetic polarisation of the inner and outer elastic membranes 750 and 700, which are provided with electrodes. In this case, the interspace 730 can be filled with a liquid (e.g. haemocompatible solution) and can be in fluid communication with (at least) one variable volume elastic bag: during the ventricular systole the electrical and/or electromagnetic control increases the volume of the interspace 730, moving the liquid from the elastic bag to the same interspace 730; during the atrial systole, the electrical and/or electromagnetic control reduces the volume of the interspace 730, moving the liquid from the latter to the elastic bag.

Moreover, the prostheses assembly of Figure 11 may also comprise one or more artificial heart valves.

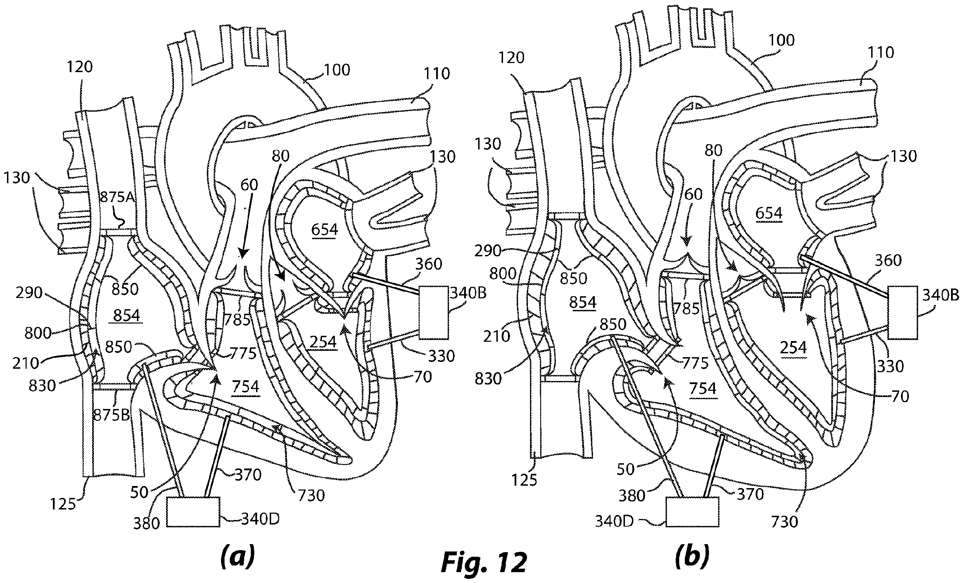

Figure 12 shows a fourth embodiment of the assembly of cardiac chamber prostheses intended for left ventricle 40, left atrium 20, right ventricle 30 and right atrium 10. In particular, the assembly comprises a right atrium prosthesis and a left atrium prosthesis, similar to those shown in Figure 9, as well as a right ventricle prosthesis, similar to the prosthesis shown in Figure 11, and a right atrium prosthesis configured to be implanted in a right atrium 10. In particular, the right atrium prosthesis of the assembly shown in Figure 12 comprises an outer elastic membrane 800 and an inner elastic membrane 850, elastically connected to each other by means of a plurality of elastic ties 290, which delimit a closed interspace 830 between them having a volume configured to be elastically variable, and wherein the outer elastic membrane 850 is configured to grip the inner wall 15 of the atrium 10 through a plurality of clips 210. The outer elastic membrane 800 and the inner elastic membrane 850 of the right atrium prosthesis are provided with a first aperture and two second apertures, the borders of which are integrally coupled to each other to form, respectively: a tricuspid valve border 885, delimiting a tricuspid valve aperture and that is configured to surround and be sutured over the native tricuspid valve 50; a superior vena cava border 875A, delimiting a superior vena cava aperture and that is configured to surround and be sutured on the outlet mouth of the superior vena cava 120; and an inferior vena cava border 875B, delimiting an inferior vena cava aperture and that is configured to surround and be sutured on the outlet mouth of the inferior vena cava 125.

In particular, for the right atrium prosthesis of Figure 12, the reference support elastic membrane structure consists of the outer elastic membrane 800, the tricuspid valve border 885 operates as outlet border, the native tricuspid valve 50 operates as native outlet valve, the superior vena cava border 875A and the inferior vena cava border 875B operate as inlet borders, the outlet mouths of the superior and inferior venae cavae 120 and 125 operate as inlet apertures of the cardiac chamber, the interspace 830 operates as primary interspace and the plurality of elastic ties 290 operates as plurality of primary variable connection elements.

Similarly to what illustrated for the left ventricle prosthesis (shown in Figure 3), the interspace 830 is configured to receive a fluid, specifically a liquid, such as a haemocompatible solution, for instance sterile saline solution, or a gas, for instance helium, in a variable amount to dynamically modify the volume of the interspace 830 itself and, thus, to modify the elastically variable volume delimited by the inner surface 854 of the inner elastic membrane 850. To this end, the interspace 830 is accessible through (at least) one access hole arranged on the outer elastic membrane 800, on which hole, after implantation of the prosthesis in the right atrium, a duct 380 is sutured, wherein the duct 380 connects the interspace 830 to an external (hydraulic or pneumatic) pump 340D that is also connected to the interspace 730 of the right ventricle prosthesis through the duct 370; alternatively, the interspace 830 could be connected to an external pump separated from the pump to which the interspace 730 of the right ventricle prosthesis is connected. Optionally, the borders of this (at least one) access hole can be made of radiopaque material and/or the surgeon can make the access hole in any position on the outer elastic membrane 800 similarly to what previously illustrated for the left ventricle prosthesis shown in Figure 3.

Also in this case, alternatively to or in combination with the hydraulic or pneumatic control, the prostheses assembly of Figure 12 can have an electrical and/or electromagnetic control carried out by an electronic processing unit configured to control the electrical and/or electromagnetic polarisation of the inner and outer elastic membranes 850 and 800, which are provided with electrodes. In this case, the interspaces 730 and 830 can be filled with a liquid (e.g. haemocompatible solution) and can be in fluid communication with each other (instead of or along with at least one variable volume elastic bag), through a communication duct: during the atrial systole, the electric and/or electromagnetic control increases the volume of the interspace 830 and reduces the volume of the interspace 730, moving the liquid from the latter to the former; during the ventricular systole, the electrical and/or electromagnetic control reduces the volume of the interspace 830 and increases the volume of the interspace 730, moving the liquid from the former to the latter.

Also, the prostheses assembly of Figure 12 may also comprise one or more artificial heart valves. In this regard, it must be noted that, in the case where the prostheses assembly is provided with an artificial tricuspid valve, the two prostheses intended for the right ventricle 30 and the right atrium 10 could be coupled to each other in correspondence of their respective tricuspid valve borders 775 and 885, and that the assembly of cardiac chamber prostheses could also be implanted without suturing the tricuspid valve border 775 and/or the tricuspid valve border 885 on the native tricuspid valve 50.

Although the embodiments of the assembly of cardiac chamber prostheses illustrated in Figures 9-12 only comprise prostheses having only an inner elastic membrane and an outer elastic membrane, it must be noted that other embodiments of the assembly of cardiac chamber prostheses according to the invention may comprise, alternatively or in combination with two-membrane prostheses, even cardiac chamber prostheses also having an intermediate elastic membrane interposed between the inner elastic membrane and the outer elastic membrane, similarly to what illustrated for the prosthesis shown in Figure 8, wherein the volume variations of the interspaces are controlled through a hydraulic or pneumatic control and/or an electrical and/or electromagnetic control. For instance, in patients with hypertrophic pathology with dilated atrium, a prostheses assembly comprising a left ventricle prosthesis having two membranes and a left atrium prosthesis having three membranes could be implanted.

It must be noted that other embodiments of the cardiac chamber prosthesis according to the invention may comprise primary (and secondary) variable connection elements, optionally elastic ones, which connect the membranes to each other, such as for instance nanotechnological structures configured to assume a configuration included between a contracted configuration, at which the connected membranes are at a first limit distance, and an expanded configuration, at which the connected membranes are at a second limit distance greater than the first limit distance. By way of example, such nanotechnological structures could comprise two bars, each of which has its own ends connected to a respective membrane, which are hinged to each other (optionally in a central area of the bars).

The cardiac chamber prosthesis, and the related assembly, according to the invention can be equipped with sensors (e.g. mechanical, piezoelectric, or electrical sensors) on the respective elastic membranes configured to detect the pressure variation and the electrocardiogram during the various phases of the cardiac cycle. In this way, adjustments of the volume variation control of the variable volume interspaces could be driven for a better overall physiological performance. For instance, inside the left ventricle, a pressure sensor can allow to process the control signal through the technique known as PRAM (described in document WO 00/64339). This processing can be used to determine the times and amplitudes of variation of the volumes delimited by the inner membranes in the specific cardiac chambers in which the related prostheses are implanted.

As stated, the cardiac chamber prosthesis, and the related assembly, according to the invention is advantageously controlled through a hydraulic or pneumatic control and/or an electric and/or electromagnetic control, advantageously through an EPROM support memory implanted as in a pacemaker. This can control either one prosthesis or more prostheses implanted in respective one or more of the four cardiac chambers.

The preferred embodiments of this invention have been described and a number of variations have been suggested hereinbefore, but it should be understood that those skilled in the art can make other variations and changes without so departing from the scope of protection thereof, as defined by the attached claims.

## Claims

1. Cardiac chamber prosthesis configured to be implanted in a cardiac chamber (10; 20; 30; 40) of a patient (440) selected from the group consisting of left ventricle (40), left atrium (20), right ventricle (30) and right atrium (10),
wherein the cardiac chamber (10; 20; 30; 40) comprises a native outlet valve (50; 60; 70; 80) and at least one inlet aperture (50; 70) selected from the group comprising a native inlet valve (50; 70) and one or more outlet mouths of venae cavae or pulmonary veins (120; 125; 130),
wherein the cardiac chamber prosthesis comprises:
- an inner elastic membrane (250; 255; 260; 650; 750; 850), having an inner surface (254; 654; 754; 854) delimiting an elastically variable volume, provided with a first aperture delimited by a respective first border and with at least one second aperture delimited by a respective second border,
- a reference support elastic membrane structure (200; 205, 225, 290A; 600; 700; 800) comprising or consisting of a outer elastic membrane (200; 205; 600; 700; 800) provided with a first aperture delimited by a respective first border and with at least one second aperture delimited by a respective second border, wherein the outer elastic membrane (200; 205; 600; 700; 800) is further provided with a plurality of clips (210) configured to grip an inner wall (45) of the cardiac chamber (10; 20; 30; 40),
wherein the borders of said first apertures of the inner and outer elastic membranes (250, 200; 255, 205; 260, 200; 650, 600; 750, 700; 850, 800) are integrally coupled to each other to form an outlet border (285; 675; 785; 885) configured to surround and be sutured on the native outlet valve (50; 60; 70; 80),
wherein the borders of said second aperture of the inner and outer elastic membranes (250, 200; 255, 205; 260, 200; 650, 600; 750, 700; 850, 800) are integrally coupled to each other to form at least one inlet border (275; 685; 775; 875A, 875B) configured to surround and be sutured on said at least one inlet aperture (50; 70),
wherein the inner elastic membrane (250; 255; 260; 650; 750; 850) and the reference support elastic membrane structure (200; 205, 225, 290A; 600; 700; 800) are connected to each other by means of a plurality of primary variable connection elements (290; 290B),
whereby the inner elastic membrane (250; 255; 260; 650; 750; 850) and the reference support elastic membrane structure (200; 205, 225, 290A; 600; 700; 800) delimit a primary interspace (230; 230B; 630; 730; 830) between them that is configured to receive a fluid with varying amount and/or pressure so as to dynamically modify a volume of the primary interspace (230; 230B; 630; 730; 830) and said elastically variable volume delimited by the inner surface (254; 654; 754; 854) of the inner elastic membrane (250; 255; 260; 650; 750; 850).

2. Cardiac chamber prosthesis according to claim 1, wherein the outer elastic membrane (200; 205; 600; 700; 800) has a coefficient of elasticity not greater, optionally lower, than a coefficient of elasticity of the inner elastic membrane (250; 255; 260; 650; 750; 850).

3. Cardiac chamber prosthesis according to claim 1 or 2, wherein each one of the inner and outer elastic membranes (250, 200; 255, 205; 260, 200; 650, 600; 750, 700; 850, 800) has a variable coefficient of elasticity, wherein each one of the inner and outer elastic membranes (250, 200; 255, 205; 260, 200; 650, 600; 750, 700; 850, 800) optionally comprises two or more areas having respective coefficients of elasticity.

4. Cardiac chamber prosthesis according to any one of the preceding claims, wherein said fluid is a liquid, optionally a haemocompatible solution, more optionally sterile saline solution, or a gas, optionally helium.

5. Cardiac chamber prosthesis according to any one of the preceding claims, further comprising at least one artificial valve (530, 540) integrally coupled to the outlet border (285; 675; 785; 885) or said at least one inlet border (275; 685; 775; 875A, 875B).

6. Cardiac chamber prosthesis according to any one of the preceding claims, wherein said primary variable connection elements are primary elastic ties (290; 290B) each one of which has a coefficient of elasticity greater than a coefficient of elasticity of the outer elastic membrane (200; 205; 600; 700; 800) and than a coefficient of elasticity of the inner elastic membrane (250; 255; 260; 650; 750; 850), wherein said primary elastic ties (290; 290B) have coefficients of elasticity optionally variable depending on the area of the inner elastic membrane (250; 255; 260; 650; 750; 850) to which they are connected.

7. Cardiac chamber prosthesis according to any one of the preceding claims, configured to be contained inside a catheter.

8. Cardiac chamber prosthesis according to any one of the preceding claims, wherein the outer elastic membrane (200; 205; 600; 700; 800) and/or the inner elastic membrane (250; 255; 260; 650; 750; 850) comprise portions which are radiopaque.

9. Cardiac chamber prosthesis according to any one of the preceding claims, wherein the primary interspace (230; 230B; 630; 730; 830) is configured to be in fluid communication with at least one variable volume elastic bag with which the prosthesis is provided or with an external pump (340A; 340B; 340C; 340D).

10. Cardiac chamber prosthesis according to any one of the preceding claims, wherein at least one between the inner elastic membrane (260) and the outer elastic membrane has a discontinuous structure, optionally selected from the group comprising:
- a honeycomb discontinuous structure formed by a plurality of elastic plates (265), more optionally hexagonal ones, connected to each other along elastic junction lines (267), wherein more optionally the elastic junction lines (267) have a coefficient of elasticity greater than a coefficient of elasticity of the elastic plates (265), and
- a discontinuous structure formed by a plurality of elastic polygonal plates, more optionally with pentagonal and/or hexagonal shape, connected to each other along elastic junction lines, wherein more optionally said elastic junction lines have a coefficient of elasticity greater than a coefficient of elasticity of said elastic polygonal plates.

11. Cardiac chamber prosthesis according to any one of the preceding claims, wherein the inner elastic membrane (250; 255; 260; 650; 750; 850) and the outer elastic membrane (200; 205; 600; 700; 800) are each provided with a plurality of electrodes configured to electrically and/or electromagnetically polarise the inner elastic membrane (250; 255; 260; 650; 750; 850) and the outer elastic membrane (200; 205; 600; 700; 800), respectively, under control of an electronic processing unit (345A; 345B) connected to said electrodes through respective control cables (206, 256).

12. Cardiac chamber prosthesis according to any one of the preceding claims, wherein the reference support elastic membrane structure (205, 225, 290A) further comprises an intermediate elastic membrane (225) that is interposed between the outer elastic membrane (205) and the inner elastic membrane (255), wherein the intermediate elastic membrane (225) optionally has coefficient of elasticity not grater, more optionally lower, than a coefficient of elasticity of the outer elastic membrane (205) and than a coefficient of elasticity of the inner elastic membrane (255),
wherein the intermediate elastic membrane (225) is provided with a first aperture delimited by a respective first border, integrally coupled to the borders of said first apertures of the inner and outer elastic membranes (255, 205), and with at least one second aperture delimited by a respective second border, integrally coupled to the borders of said second apertures of the inner and outer elastic membranes (255, 205),
wherein the intermediate elastic membrane (225) and the outer elastic membrane (205) are connected to each other by means of a plurality of secondary variable connection elements (290A), optionally consisting of secondary elastic ties (290A) each one of which more optionally has a coefficient of elasticity greater than the coefficient of elasticity of the outer elastic membrane (205) and than the coefficient of elasticity of the intermediate elastic membrane (225), wherein said secondary elastic ties (290A) have coefficients of elasticity variable depending on the area of the intermediate elastic membrane (225) to which they are connected, whereby said plurality of primary variable connection elements (290B) connect the inner elastic membrane (255) and the intermediate elastic membrane (225) to each other,
whereby the outer elastic membrane (205) and the intermediate elastic membrane (225) delimit a secondary interspace (230A) between them that is configured to receive a fluid, optionally consisting in a liquid, more optionally a haemocompatible solution, still more optionally sterile saline solution, or in a gas, optionally helium.

13. Cardiac chamber prosthesis according to claim 12, wherein the secondary interspace (230A) is configured to receive said fluid with varying amount and/or pressure, so as to dynamically modify a volume of the secondary interspace (230A), wherein the secondary interspace (230A) is optionally configured to be in fluid communication with at least one variable volume elastic bag with which the prosthesis is provided or with an external pump (340A; 340B; 340C; 340D).

14. Cardiac chamber prosthesis according to claim 12 or 13, wherein the intermediate elastic membrane (225) has a discontinuous structure, optionally selected from the group comprising:
- a honeycomb discontinuous structure formed by a plurality of elastic plates, more optionally hexagonal ones, connected to each other along elastic junction lines, wherein more optionally said elastic junction lines have a coefficient of elasticity greater than a coefficient of elasticity of said elastic plates, and
- a discontinuous structure formed by a plurality of elastic polygonal plates, more optionally with pentagonal and/or hexagonal shape, connected to each other along elastic junction lines, wherein more optionally said elastic junction lines have a coefficient of elasticity greater than a coefficient of elasticity of said elastic polygonal plates.

15. Cardiac chamber prosthesis according to any one of claims 12 to 14, when depending on claim 11, wherein the intermediate elastic membrane (225) is provided with a plurality of electrodes configured to electrically and/or electromagnetically polarise the intermediate elastic membrane (225) under control of said electronic processing unit (345B) connected to said electrodes through respective one or more control cables (226).

16. Cardiac chamber prosthesis according to any one of the preceding claims, wherein the cardiac chamber prosthesis is configured to be implanted:
- in a left ventricle (40), whereby the outlet border is an aortic border (285) configured to surround and be sutured on a native aortic valve (80) and said at least one inlet border consists of a mitral border (275) configured to surround and be sutured on a native mitral valve (70), or
- in a left atrium (20), whereby the outlet border is a mitral border (675) configured to surround and be sutured on a native mitral valve (70) and said at least one inlet border consists of one or more borders (685) of pulmonary veins configured to surround and be sutured in correspondence of respective one or more outlet mouths of pulmonary veins (130), or
- in a right ventricle (30), whereby the outlet border is a pulmonary valve border (785) configured to surround and be sutured on a native pulmonary valve (60) and said at least one inlet border consists of a tricuspid valve border (775) configured to surround and be sutured on a native tricuspid valve (50), or
- in a right atrium (10), whereby the outlet border is a tricuspid valve border (775) configured to surround and be sutured on a native tricuspid valve (50) and said at least one inlet border consists of one or more borders (875A, 875B) of venae cavae configured to surround and be sutured in correspondence of respective one or more outlet mouths of venae cavae (120, 125).

17. Assembly of cardiac chamber prostheses comprising two to four cardiac chamber prostheses according to any one of claims 1 to 16, wherein said cardiac chamber prostheses are configured to be implanted in respective two or more cardiac chambers (10; 20; 30; 40) of a patient (440).

18. Assembly of cardiac chamber prostheses according to claim 17, wherein said cardiac chamber prostheses comprise or consist of two cardiac chamber prostheses according to claim 11 or according to any one of claims 12 to 16, when depending on claim 11, which are associated to each other, which are configured to be implanted in a left ventricle (40) and in a left atrium (20), or in a right ventricle (30) and in a right atrium (10), wherein the primary interspaces (230, 630; 730, 830) of the associated two cardiac chamber prostheses are in fluid communication with each other through a communication duct, whereby the volume of the primary interspace (230; 730) of one of the two associated cardiac chamber prostheses is configured to increase when the volume of the primary interspace (630; 830) of the other one of the two associated cardiac chamber prostheses decreases, and vice versa, through a displacement of fluid between the primary interspaces (230, 630; 730, 830) of the two associated cardiac chamber prostheses.

19. Cardiac assistance system comprising:
- a cardiac chamber prosthesis according to any one of claims 1 to 16 or an assembly of cardiac chamber prostheses according to claim 17 or 18,
- at least one pump (340A; 340B; 340C; 340D) and/or an electronic processing unit (345A; 345B) configured to control an amount and/or a pressure of said fluid received in the primary interspace (230; 230B; 630; 730; 830) of said cardiac chamber prosthesis or of the cardiac chamber prostheses of said assembly of cardiac chamber prostheses so as to dynamically modify the volume of the primary interspace (230; 230B; 630; 730; 830) of said cardiac chamber prosthesis or of the cardiac chamber prostheses of said assembly of cardiac chamber prostheses.

## Patentansprüche

1. Herzkammerprothese, die so konfiguriert ist, dass sie in eine Herzkammer (10; 20; 30; 40) eines Patienten (440), ausgewählt aus der Gruppe bestehend aus linker Herzkammer (40), linkem Vorhof (20), rechter Herzkammer (30) und rechtem Vorhof (10), implantiert werden kann,
wobei die Herzkammer (10; 20; 30; 40) ein natives Auslassventil (50; 60; 70; 80) und mindestens eine Einlassöffnung (50; 70) umfasst, die aus der Gruppe ausgewählt ist, die ein natives Einlassventil (50; 70) und eine oder mehrere Auslassmündungen von Venae cavae oder Lungenvenen (120; 125; 130) umfasst, wobei die Herzkammerprothese umfasst:
- eine innere elastische Membran (250; 255; 260; 650; 750; 850) mit einer inneren Oberfläche (254; 654; 754; 854), die ein elastisch veränderliches Volumen begrenzt, das mit einer ersten Öffnung, die durch einen jeweiligen ersten Rand begrenzt ist, und mit mindestens einer zweiten Öffnung, die durch einen jeweiligen zweiten Rand begrenzt ist, versehen ist,
- eine elastische Referenz-Stützmembranstruktur (200; 205, 225, 290A; 600; 700; 800), die eine äußere elastische Membran (200; 205; 600; 700; 800) umfasst oder aus dieser besteht, die mit einer ersten Öffnung, die durch einen jeweiligen ersten Rand begrenzt ist, und mit mindestens einer zweiten Öffnung, die durch einen jeweiligen zweiten Rand begrenzt ist, versehen ist, wobei die äußere elastische Membran (200; 205; 600; 700; 800) ferner mit einer Vielzahl von Clips (210) versehen ist, die so konfiguriert sind, dass sie eine Innenwand (45) der Herzkammer (10; 20; 30; 40) greifen,
wobei die Ränder der ersten Öffnungen der inneren und äußeren elastischen Membranen (250, 200; 255, 205; 260, 200; 650, 600; 750, 700; 850, 800) einstückig miteinander verbunden sind, um einen Auslassrand (285; 675; 785; 885) zu bilden, der so konfiguriert ist, dass er das native Auslassventil (50; 60; 70; 80) umgibt und daran angenäht wird,
wobei die Ränder der zweiten Öffnung der inneren und äußeren elastischen Membranen (250, 200; 255, 205; 260, 200; 650, 600; 750, 700; 850, 800) einstückig miteinander verbunden sind, um mindestens einen Einlassrand (275; 685; 775; 875A, 875B) zu bilden, der so konfiguriert ist, dass er die mindestens eine Einlassöffnung (50; 70) zu umgeben und daran angenäht zu werden,
wobei die innere elastische Membran (250; 255; 260; 650; 750; 850) und die elastische Referenz-Stützmembranstruktur (200; 205, 225, 290A; 600; 700; 800) mittels einer Vielzahl miteinander verbunden sind von primären variablen Verbindungselementen (290; 290B),
wobei die innere elastische Membran (250; 255; 260; 650; 750; 850) und die elastische Referenz-Stützmembranstruktur (200; 205, 225, 290A; 600; 700; 800) einen primären Zwischenraum (230; 230B; 630; 730; 830) begrenzen, der so konfiguriert ist, dass er ein Fluid mit variierender Menge und/oder variierendem Druck aufnehmen kann, um ein Volumen des primären Zwischenraums (230; 230B; 630; 730; 830) und des elastisch variablen Volumens, das durch die innere Oberfläche (254; 654; 754; 854) der inneren elastischen Membran (250; 255; 260; 650; 750; 850) begrenzt ist, dynamisch zu verändern.

2. Herzkammerprothese nach Anspruch 1, wobei die äußere elastische Membran (200; 205; 600; 700; 800) einen Elastizitätskoeffizienten aufweist, der nicht größer, gegebenenfalls geringer, als ein Elastizitätskoeffizient der inneren elastischen Membran (250; 255; 260; 650; 750; 850) ist.

3. Herzkammerprothese nach Anspruch 1 oder 2, wobei jede der inneren und äußeren elastischen Membranen (250, 200; 255, 205; 260, 200; 650, 600; 750, 700; 850, 800) einen variablen Elastizitätskoeffizienten aufweist, wobei jede der inneren und äußeren elastischen Membranen (250, 200; 255, 205; 260, 200; 650, 600; 750, 700; 850, 800) optional zwei oder mehr Bereiche mit entsprechenden Elastizitätskoeffizienten umfasst.

4. Herzkammerprothese nach einem der vorhergehenden Ansprüche, wobei das Fluid eine Flüssigkeit, gegebenenfalls eine hämokompatible Lösung, gegebenenfalls eine sterile Kochsalzlösung, oder ein Gas, gegebenenfalls Helium, ist.

5. Herzkammerprothese nach einem der vorhergehenden Ansprüche, die ferner mindestens ein künstliches Ventil (530, 540) umfasst, das integral mit dem Auslassrand (285; 675; 785; 885) oder dem mindestens einen Einlassrand (275; 685; 775; 875A, 875B) verbunden ist.

6. Herzkammerprothese nach einem der vorhergehenden Ansprüche, wobei die primären variablen Verbindungselemente primäre elastische Bänder (290; 290B) sind, von denen jedes einen Elastizitätskoeffizienten aufweist, der größer ist als ein Elastizitätskoeffizient der äußeren elastischen Membran (200; 205; 600; 700; 800) und als ein Elastizitätskoeffizient der inneren elastischen Membran (250; 255; 260; 650; 750; 850) ist, wobei die primären elastischen Bänder (290; 290B) Elastizitätskoeffizienten aufweisen, die optional variabel sind in Abhängigkeit von der Fläche der inneren elastischen Membran (250; 255; 260; 650; 750; 850), mit der sie verbunden sind.

7. Herzkammerprothese nach einem der vorhergehenden Ansprüche, die so gestaltet ist, dass sie in einem Katheter untergebracht werden kann.

8. Herzkammerprothese nach einem der vorhergehenden Ansprüche, wobei die äußere elastische Membran (200; 205; 600; 700; 800) und/oder die innere elastische Membran (250; 255; 260; 650; 750; 850) Teile aufweisen, die röntgendicht sind.

9. Herzkammerprothese nach einem der vorhergehenden Ansprüche, wobei der primäre Zwischenraum (230; 230B; 630; 730; 830) so konfiguriert ist, dass er in Fluidverbindung mit mindestens einem elastischen Beutel mit variablem Volumen, mit dem die Prothese versehen ist, oder mit einer externen Pumpe (340A; 340B; 340C; 340D) steht.

10. Herzkammerprothese nach einem der vorhergehenden Ansprüche, wobei mindestens eine zwischen der inneren elastischen Membran (260) und der äußeren elastischen Membran eine diskontinuierliche Struktur aufweist, wahlweise ausgewählt aus der Gruppe umfassend:
- eine wabenförmige diskontinuierliche Struktur, die durch eine Vielzahl von elastischen Platten (265), gegebenenfalls hexagonalen, gebildet wird, die entlang elastischer Verbindungslinien (267) miteinander verbunden sind, wobei gegebenenfalls die elastischen Verbindungslinien (267) einen Elastizitätskoeffizienten aufweisen, der größer ist als ein Elastizitätskoeffizient der elastischen Platten (265), und
- eine diskontinuierliche Struktur, die aus einer Vielzahl von elastischen polygonalen Platten besteht, optional mit fünfeckiger und/oder sechseckiger Form, die entlang elastischer Verbindungslinien miteinander verbunden sind, wobei die elastischen Verbindungslinien einen größeren Elastizitätskoeffizienten als der Elastizitätskoeffizient der elastischen polygonalen Platten aufweisen.

11. Herzkammerprothese nach einem der vorhergehenden Ansprüche, wobei die innere elastische Membran (250; 255; 260; 650; 750; 850) und die äußere elastische Membran (200; 205; 600; 700; 800) jeweils mit einer Vielzahl von Elektroden versehen sind, die so konfiguriert sind, dass sie die innere elastische Membran (250; 255; 260; 650; 750; 850) bzw. die äußere elastische Membran (200; 205; 600; 700; 800) unter der Steuerung einer elektronischen Verarbeitungseinheit (345A; 3458) elektrisch und/oder elektromagnetisch polarisieren, die mit den Elektroden über jeweilige Steuerkabel (206, 256) verbunden ist.

12. Herzkammerprothese nach einem der vorhergehenden Ansprüche, wobei die elastische Referenz-Stützmembranstruktur (205, 225, 290A) ferner eine elastische Zwischenmembran (225) umfasst, die zwischen der äußeren elastischen Membran (205) und der inneren elastischen Membran (255) angeordnet ist, wobei die elastische Zwischenmembran (225) optional einen Elastizitätskoeffizienten aufweist, der nicht größer, sondern optional niedriger als ein Elastizitätskoeffizient der äußeren elastischen Membran (205) und als ein Elastizitätskoeffizient der inneren elastischen Membran (255) ist,
wobei die elastische Zwischenmembran (225) mit einer ersten Öffnung versehen ist, die durch einen jeweiligen ersten Rand begrenzt ist, der integral mit den Rändern der ersten Öffnungen der inneren und äußeren elastischen Membranen (255, 205) verbunden ist, und mit mindestens einer zweiten Öffnung, die durch einen jeweiligen zweiten Rand begrenzt ist, der integral mit den Rändern der zweiten Öffnungen der inneren und äußeren elastischen Membranen (255, 205) verbunden ist,
wobei die elastische Zwischenmembran (225) und die äußere elastische Membran (205) miteinander mittels einer Vielzahl von sekundären variablen Verbindungselementen (290A) verbunden sind, die optional aus sekundären elastischen Bändern (290A) bestehen, von denen jedes optional einen Elastizitätskoeffizienten aufweist, der größer ist als der Elastizitätskoeffizient der äußeren elastischen Membran (205) und als der Elastizitätskoeffizient der elastischen Zwischenmembran (225), wobei die sekundären elastischen Verbindungen (290A) Elastizitätskoeffizienten aufweisen, die in Abhängigkeit von der Fläche der elastischen Zwischenmembran (225), mit der sie verbunden sind, variabel sind, wobei die Vielzahl der primären variablen Verbindungselemente (290B) die innere elastische Membran (255) und die elastische Zwischenmembran (225) miteinander verbinden,
wobei die äußere elastische Membran (205) und die elastische Zwischenmembran (225) einen sekundären Zwischenraum (230A) zwischen sich begrenzen, der so konfiguriert ist, dass er ein Fluid aufnehmen kann, das optional aus einer Flüssigkeit, optionaler aus einer hämokompatiblen Lösung, noch optionaler aus einer sterilen Kochsalzlösung, oder aus einem Gas, optional Helium, besteht.

13. Herzkammerprothese nach Anspruch 12, wobei der sekundäre Zwischenraum (230A) so konfiguriert ist, dass er das Fluid mit variierender Menge und/oder variierendem Druck aufnimmt, um ein Volumen des sekundären Zwischenraums (230A) dynamisch zu modifizieren, wobei der sekundäre Zwischenraum (230A) optional so konfiguriert ist, dass er in Fluidverbindung mit mindestens einem elastischen Beutel mit variablem Volumen, mit dem die Prothese versehen ist, oder mit einer externen Pumpe (340A; 340B; 340C; 340D) steht.

14. Herzkammerprothese nach Anspruch 12 oder 13, wobei die elastische Zwischenmembran (225) eine diskontinuierliche Struktur aufweist, die wahlweise aus der Gruppe ausgewählt ist, die Folgendes umfasst:
- eine diskontinuierliche Wabenstruktur, die aus einer Vielzahl von elastischen, gegebenenfalls sechseckigen Platten besteht, die entlang elastischer Verbindungslinien miteinander verbunden sind, wobei gegebenenfalls die elastischen Verbindungslinien einen Elastizitätskoeffizienten aufweisen, der größer ist als der Elastizitätskoeffizient der elastischen Platten, und
- eine diskontinuierliche Struktur, die aus einer Vielzahl von elastischen polygonalen Platten besteht, optional mit fünfeckiger und/oder sechseckiger Form, die entlang elastischer Verbindungslinien miteinander verbunden sind, wobei die elastischen Verbindungslinien einen größeren Elastizitätskoeffizienten als der Elastizitätskoeffizient der elastischen polygonalen Platten aufweisen.

15. Herzkammerprothese nach einem der Ansprüche 12 bis 14, wenn sie von Anspruch 11 abhängt, wobei die elastische Zwischenmembran (225) mit einer Vielzahl von Elektroden versehen ist, die so konfiguriert sind, dass sie die elastische Zwischenmembran (225) unter der Steuerung der elektronischen Verarbeitungseinheit (345B) elektrisch und/oder elektromagnetisch polarisieren, die mit den Elektroden über jeweilige ein oder mehrere Steuerkabel (226) verbunden ist.

16. Herzkammerprothese nach einem der vorhergehenden Ansprüche, wobei die Herzkammerprothese zur Implantation konfiguriert ist:
- in einem linken Ventrikel (40), wobei der Auslassrand ein Aortenrand (285) ist, der so konfiguriert ist, dass er eine native Aortenklappe (80) umgibt und mit ihr vernäht wird, und der mindestens eine Einlassrand aus einem Mitralrand (275) besteht, der so konfiguriert ist, dass er eine native Mitralklappe (70) umgibt und mit ihr vernäht wird, oder
- in einem linken Atrium (20), wobei der Auslassrand ein Mitralrand (675) ist, der so konfiguriert ist, dass er eine native Mitralklappe (70) umgibt und mit ihr vernäht wird, und der mindestens eine Einlassrand aus einem oder mehreren Rändern (685) von Pulmonalvenen besteht, die so konfiguriert sind, dass sie jeweils eine oder mehrere Auslassmündungen von Pulmonalvenen (130) umgeben und mit ihnen vernäht werden, oder
- in einem rechten Ventrikel (30), wobei der Auslassrand ein Pulmonalklappenrand (785) ist, der so konfiguriert ist, dass er eine native Pulmonalklappe (60) umgibt und mit ihr vernäht wird, und der mindestens eine Einlassrand aus einem Trikuspidalklappenrand (775) besteht, der so konfiguriert ist, dass er eine native Trikuspidalklappe (50) umgibt und mit ihr vernäht wird, oder
- in einem rechten Atrium (10), wobei der Auslassrand ein Trikuspidalklappenrand (775) ist, der so konfiguriert ist, dass er eine native Trikuspidalklappe (50) umgibt und mit ihr vernäht wird, und der mindestens eine Einlassrand aus einem oder mehreren Rändern (875A, 875B) von Venae cavae besteht, die so konfiguriert sind, dass sie eine oder mehrere Auslassöffnungen von Venae cavae (120, 125) umgeben und mit ihnen vernäht werden.

17. Anordnung von Herzkammerprothesen, die zwei bis vier Herzkammerprothesen nach einem der Ansprüche 1 bis 16 umfasst, wobei die Herzkammerprothesen so konfiguriert sind, dass sie in jeweils zwei oder mehr Herzkammern (10; 20; 30; 40) eines Patienten (440) implantiert werden.

18. Anordnung von Herzkammerprothesen nach Anspruch 17, wobei die Herzkammerprothesen zwei Herzkammerprothesen nach Anspruch 11 oder nach einem der Ansprüche 12 bis 16, wenn sie von Anspruch 11 abhängen, umfassen oder daraus bestehen, die einander zugeordnet sind, die so konfiguriert sind, dass sie in eine linke Herzkammer (40) und in ein linkes Atrium (20) oder in eine rechte Herzkammer (30) und in ein rechtes Atrium (10) implantiert werden können, wobei die primären Zwischenräume (230, 630; 730, 830) der beiden zugehörigen Herzkammerprothesen durch einen Verbindungskanal in Fluidverbindung miteinander stehen, wobei das Volumen des primären Zwischenraums (230; 730) einer der beiden zugehörigen Herzkammerprothesen so konfiguriert ist, dass es sich vergrößert, wenn sich das Volumen des primären Zwischenraums (630; 830) der anderen der beiden zugehörigen Herzkammerprothesen verringert, und umgekehrt, durch eine Verschiebung von Fluid zwischen den primären Zwischenräumen (230, 630; 730, 830) der beiden zugehörigen Herzkammerprothesen.

19. Herzunterstützungssystem umfassend:
- eine Herzkammerprothese nach einem der Ansprüche 1 bis 16 oder eine Anordnung von Herzkammerprothesen nach Anspruch 17 oder 18,
- mindestens eine Pumpe (340A; 340B; 340C; 340D) und/oder eine elektronische Verarbeitungseinheit (345A; 345B), die so konfiguriert ist, dass sie eine Menge und/oder einen Druck des in dem primären Zwischenraum (230; 230B; 630; 730; 830) der Herzkammerprothese oder der Herzkammerprothesen der Anordnung von Herzkammerprothesen zu steuern, um das Volumen des primären Zwischenraums (230; 230B; 630; 730; 830) der Herzkammerprothese oder der Herzkammerprothesen der Anordnung von Herzkammerprothesen dynamisch zu verändern.

## Revendications

1. Prothèse de chambre cardiaque configurée pour être implantée dans une chambre cardiaque (10 ; 20 ; 30 ; 40) d'un patient (440) choisie dans le groupe constitué du ventricule gauche (40), de l'oreillette gauche (20), du ventricule droit (30) et de l'oreillette droite (10),
dans laquelle la chambre cardiaque (10 ; 20 ; 30 ; 40) comprend une valvule de sortie native (50 ; 60 ; 70 ; 80) et au moins une ouverture d'entrée (50 ; 70) choisie dans le groupe comprenant une valvule d'entrée native (50 ; 70) et une ou plusieurs embouchures de sortie de veines caves ou de veines pulmonaires (120 ; 125 ; 130),
dans laquelle la prothèse de chambre cardiaque comprend :
- une membrane élastique interne (250 ; 255 ; 260 ; 650 ; 750 ; 850), ayant une surface interne (254 ; 654 ; 754 ; 854) délimitant un volume élastiquement variable, munie d'une première ouverture délimitée par une première bordure respective et d'au moins une deuxième ouverture délimitée par une deuxième bordure respective,
- une structure de membrane élastique de support de référence (200 ; 205, 225, 290A ; 600 ; 700 ; 800) comprenant ou constituée par une membrane élastique externe (200 ; 205 ; 600 ; 700 ; 800) munie d'une première ouverture délimitée par une première bordure respective et d'au moins une deuxième ouverture délimitée par une deuxième bordure respective, où la membrane élastique externe (200 ; 205 ; 600 ; 700 ; 800) est en outre munie d'une pluralité de clips (210) configurés pour saisir une paroi interne (45) de la chambre cardiaque (10 ; 20 ; 30 ; 40),
dans laquelle les bordures desdites premières ouvertures des membranes élastiques interne et externe (250, 200 ; 255, 205 ; 260, 200 ; 650, 600 ; 750, 700 ; 850, 800) sont intégralement couplées entre elles pour former une bordure de sortie (285 ; 675 ; 785 ; 885) configurée pour entourer et être suturée sur la valvule de sortie native (50 ; 60 ; 70 ; 80),
dans laquelle les bordures de ladite deuxième ouverture des membranes élastiques interne et externe (250, 200 ; 255, 205 ; 260, 200 ; 650, 600 ; 750, 700 ; 850, 800) sont intégralement couplées entre elles pour former au moins une bordure d'entrée (275 ; 685 ; 775 ; 875A, 875B) configurée pour entourer et être suturée sur ladite au moins une ouverture d'entrée (50 ; 70),
dans laquelle la membrane élastique interne (250 ; 255 ; 260 ; 650 ; 750 ; 850) et la structure de membrane élastique de support de référence (200 ; 205, 225, 290A ; 600 ; 700 ; 800) sont reliées entre elles au moyen d'une pluralité d'éléments de liaison variables primaires (290 ; 290B),
moyennant quoi la membrane élastique interne (250 ; 255 ; 260 ; 650 ; 750 ; 850) et la structure de membrane élastique de support de référence (200 ; 205, 225, 290A ; 600 ; 700 ; 800) délimitent entre elles un espace intermédiaire primaire (230 ; 230B ; 630 ; 730 ; 830) qui est configuré pour recevoir un fluide avec une quantité et/ou une pression variable(s) de manière à modifier dynamiquement un volume de l'espace intermédiaire primaire (230 ; 230B ; 630 ; 730 ; 830) et ledit volume élastiquement variable délimité par la surface interne (254 ; 654 ; 754 ; 854) de la membrane élastique interne (250 ; 255 ; 260 ; 650 ; 750 ; 850).

2. Prothèse de chambre cardiaque selon la revendication 1, dans laquelle la membrane élastique externe (200 ; 205 ; 600 ; 700 ; 800) a un coefficient d'élasticité qui n'est pas supérieur, éventuellement inférieur, à un coefficient d'élasticité de la membrane élastique interne (250 ; 255 ; 260 ; 650 ; 750 ; 850).

3. Prothèse de chambre cardiaque selon la revendication 1 ou 2, dans laquelle chacune des membranes élastiques interne et externe (250, 200 ; 255, 205 ; 260, 200 ; 650, 600 ; 750, 700 ; 850, 800) a un coefficient d'élasticité variable, où chacune des membranes élastiques interne et externe (250, 200 ; 255, 205 ; 260, 200 ; 650, 600 ; 750, 700 ; 850, 800) comprend éventuellement deux zones ou plus ayant des coefficients d'élasticité respectifs.

4. Prothèse de chambre cardiaque selon l'une quelconque des revendications précédentes, dans laquelle ledit fluide est un liquide, éventuellement une solution hémocompatible, plus éventuellement une solution saline stérile, ou un gaz, éventuellement de l'hélium.

5. Prothèse de chambre cardiaque selon l'une quelconque des revendications précédentes, comprenant en outre au moins une valvule artificielle (530, 540) intégralement couplée à la bordure de sortie (285 ; 675 ; 785 ; 885) ou à ladite au moins une bordure d'entrée (275 ; 685 ; 775 ; 875A, 875B).

6. Prothèse de chambre cardiaque selon l'une quelconque des revendications précédentes, dans laquelle lesdits éléments de liaison variables primaires sont des liens élastiques primaires (290 ; 290B) dont chacun a un coefficient d'élasticité supérieur à un coefficient d'élasticité de la membrane élastique externe (200 ; 205 ; 600 ; 700 ; 800) et à un coefficient d'élasticité de la membrane élastique interne (250 ; 255 ; 260 ; 650 : 750 ; 850), où lesdits liens élastiques primaires (290 ; 290B) ont des coefficients d'élasticité éventuellement variables selon la zone de la membrane élastique interne (250 ; 255 ; 260 ; 650 ; 750 ; 850) à laquelle ils sont reliés.

7. Prothèse de chambre cardiaque selon l'une quelconque des revendications précédentes, configurée pour être contenue à l'intérieur d'un cathéter.

8. Prothèse de chambre cardiaque selon l'une quelconque des revendications précédentes, dans laquelle la membrane élastique externe (200 ; 205 ; 600 ; 700 ; 800) et/ou la membrane élastique interne (250 ; 255 ; 260 ; 650 ; 750 ; 850) comprend/comprennent des parties qui sont radio-opaques.

9. Prothèse de chambre cardiaque selon l'une quelconque des revendications précédentes, dans laquelle l'espace intermédiaire primaire (230 ; 230B ; 630 ; 730 ; 830) est configuré pour être en communication fluidique avec au moins un sac élastique à volume variable dont est munie la prothèse ou avec une pompe externe (340A ; 340B ; 340C ; 340D).

10. Prothèse de chambre cardiaque selon l'une quelconque des revendications précédentes, dans laquelle au moins l'une entre la membrane élastique interne (260) et la membrane élastique externe a une structure discontinue, éventuellement choisie dans le groupe comprenant :
- une structure discontinue en nid d'abeille formée par une pluralité de plaques élastiques (265), plus éventuellement de plaques hexagonales, reliées entre elles le long de lignes de jonction élastiques (267), où plus éventuellement les lignes de jonction élastiques (267) ont un coefficient d'élasticité supérieur à un coefficient d'élasticité des plaques élastiques (265), et
- une structure discontinue formée par une pluralité de plaques polygonales élastiques, plus éventuellement de forme pentagonale et/ou hexagonale, reliées entre elles le long de lignes de jonction élastiques, où plus éventuellement lesdites lignes de jonction élastiques ont un coefficient d'élasticité supérieur à un coefficient d'élasticité desdites plaques polygonales élastiques.

11. Prothèse de chambre cardiaque selon l'une quelconque des revendications précédentes, dans laquelle la membrane élastique interne (250 ; 255 ; 260 ; 650 ; 750 ; 850) et la membrane élastique externe (200 ; 205 ; 600 ; 700 ; 800) sont chacune munies d'une pluralité d'électrodes configurées pour polariser électriquement et/ou électromagnétiquement la membrane élastique interne (250 ; 255 ; 260 ; 650 ; 750 ; 850) et la membrane élastique externe (200 ; 205 ; 600 ; 700 ; 800), respectivement, sous le contrôle d'une unité de traitement électronique (345A ; 345B) reliée auxdites électrodes par des câbles de commande respectifs (206, 256).

12. Prothèse de chambre cardiaque selon l'une quelconque des revendications précédentes, dans laquelle la structure de membrane élastique de support de référence (205, 225, 290A) comprend en outre une membrane élastique intermédiaire (225) qui est interposée entre la membrane élastique externe (205) et la membrane élastique interne (255), où la membrane élastique intermédiaire (225) a éventuellement un coefficient d'élasticité qui n'est pas supérieur, plus éventuellement inférieur, à un coefficient d'élasticité de la membrane élastique externe (205) et à un coefficient d'élasticité de la membrane élastique interne (255),
dans laquelle la membrane élastique intermédiaire (225) est munie d'une première ouverture délimitée par une première bordure respective, intégralement couplée aux bordures desdites premières ouvertures des membranes élastiques interne et externe (255, 205), et d'au moins une deuxième ouverture délimitée par une deuxième bordure respective, intégralement couplée aux bordures desdites deuxièmes ouvertures des membranes élastiques interne et externe (255, 205),
dans laquelle la membrane élastique intermédiaire (225) et la membrane élastique externe (205) sont reliées entre elles au moyen d'une pluralité d'éléments de liaison variables secondaires (290A), éventuellement constitués de liens élastiques secondaires (290A) dont chacun plus éventuellement a un coefficient d'élasticité supérieur au coefficient d'élasticité de la membrane élastique externe (205) et au coefficient d'élasticité de la membrane élastique intermédiaire (225), où lesdits liens élastiques secondaires (290A) ont des coefficients d'élasticité variables selon la zone de la membrane élastique intermédiaire (225) à laquelle ils sont reliés,
moyennant quoi ladite pluralité d'éléments de liaison variables primaires (290B) relient la membrane élastique interne (255) et la membrane élastique intermédiaire (225) l'une à l'autre,
moyennant quoi la membrane élastique externe (205) et la membrane élastique intermédiaire (225) délimitent entre elles un espace intermédiaire secondaire (230A) qui est configuré pour recevoir un fluide, éventuellement consistant en un liquide, plus éventuellement une solution hémocompatible, encore plus éventuellement une solution saline stérile, ou un gaz, éventuellement de l'hélium.

13. Prothèse de chambre cardiaque selon la revendication 12, dans laquelle l'espace intermédiaire secondaire (230A) est configuré pour recevoir ledit fluide avec une quantité et/ou une pression variable(s), de manière à modifier dynamiquement un volume de l'espace intermédiaire secondaire (230A), où l'espace intermédiaire secondaire (230A) est éventuellement configuré pour être en communication fluidique avec au moins un sac élastique à volume variable dont est munie la prothèse ou avec une pompe externe (340A ; 340B ; 340C ; 340D).

14. Prothèse de chambre cardiaque selon la revendication 12 ou 13, dans laquelle la membrane élastique intermédiaire (225) a une structure discontinue, éventuellement choisie dans le groupe comprenant :
- une structure discontinue en nid d'abeille formée par une pluralité de plaques élastiques, plus éventuellement de plaques hexagonales, reliées entre elles le long de lignes de jonction élastiques, où plus éventuellement lesdites lignes de jonction élastiques ont un coefficient d'élasticité supérieur à un coefficient d'élasticité desdites plaques élastiques, et
- une structure discontinue formée par une pluralité de plaques polygonales élastiques, plus éventuellement de forme pentagonale et/ou hexagonale, reliées entre elles le long de lignes de jonction élastiques, où plus éventuellement lesdites lignes de jonction élastiques ont un coefficient d'élasticité supérieur à un coefficient d'élasticité desdites plaques polygonales élastiques.

15. Prothèse de chambre cardiaque selon l'une quelconque des revendications 12 à 14, lorsqu'elles dépendent de la revendication 11, dans laquelle la membrane élastique intermédiaire (225) est munie d'une pluralité d'électrodes configurées pour polariser électriquement et/ou électromagnétiquement la membrane élastique intermédiaire (225) sous le contrôle de ladite unité de traitement électronique (345B) reliée auxdites électrodes par un ou plusieurs câbles de commande respectifs (226).

16. Prothèse de chambre cardiaque selon l'une quelconque des revendications précédentes, dans laquelle la prothèse de chambre cardiaque est configurée pour être implantée :
- dans un ventricule gauche (40), moyennant quoi la bordure de sortie est une bordure aortique (285) configurée pour entourer et être suturée sur une valvule aortique native (80) et ladite au moins une bordure d'entrée est constituée d'une bordure mitrale (275) configurée pour entourer et être suturée sur une valvule mitrale native (70), ou
- dans une oreillette gauche (20), moyennant quoi la bordure de sortie est une bordure mitrale (675) configurée pour entourer et être suturée sur une valvule mitrale native (70) et ladite au moins une bordure d'entrée est constituée d'une ou de plusieurs bordures (685) de veines pulmonaires configurées pour entourer et être suturées en correspondance avec une ou plusieurs embouchures de sortie respectives de veines pulmonaires (130), ou
- dans un ventricule droit (30), moyennant quoi la bordure de sortie est une bordure de valvule pulmonaire (785) configurée pour entourer et être suturée sur une valvule pulmonaire native (60) et ladite au moins une bordure d'entrée est constituée d'une bordure de valvule tricuspide (775) configurée pour entourer et être suturée sur une valvule tricuspide native (50), ou
- dans une oreillette droite (10), moyennant quoi la bordure de sortie est une bordure de valvule tricuspide (775) configurée pour entourer et être suturée sur une valvule tricuspide native (50) et ladite au moins une bordure d'entrée est constituée d'une ou de plusieurs bordures (875A, 875B) de veines caves configurées pour entourer et être suturées en correspondance avec une ou plusieurs embouchures de sortie respectives de veines caves (120, 125).

17. Ensemble de prothèses de chambre cardiaque comprenant deux à quatre prothèses de chambre cardiaque selon l'une quelconque des revendications 1 à 16, dans lequel lesdites prothèses de chambre cardiaque sont configurées pour être implantées dans deux chambres cardiaques respectives (10 ; 20 ; 30 ; 40) ou plus d'un patient (440).

18. Ensemble de prothèses de chambre cardiaque selon la revendication 17, dans lequel lesdites prothèses de chambre cardiaque comprennent ou sont constituées par deux prothèses de chambre cardiaque selon la revendication 11 ou selon l'une quelconque des revendications 12 à 16, lorsqu'elles dépendent de la revendication 11, qui sont associées l'une à l'autre, qui sont configurées pour être implantées dans un ventricule gauche (40) et dans une oreillette gauche (20), ou dans un ventricule droit (30) et dans une oreillette droite (10), où les espaces intermédiaires primaires (230, 630 ; 730, 830) des deux prothèses de chambre cardiaque associées sont en communication fluidique l'un avec l'autre par un conduit de communication, moyennant quoi le volume de l'espace intermédiaire primaire (230 ; 730) de l'une des deux prothèses de chambre cardiaque associées est configuré pour augmenter lorsque le volume de l'espace intermédiaire primaire (630 ; 830) de l'autre des deux prothèses de chambre cardiaque associées diminue, et vice versa, par un déplacement de fluide entre les espaces intermédiaires primaires (230, 630 ; 730, 830) des deux prothèses de chambre cardiaque associées.

19. Système d'assistance cardiaque comprenant :
- une prothèse de chambre cardiaque selon l'une quelconque des revendications 1 à 16 ou un ensemble de prothèses de chambre cardiaque selon la revendication 17 ou 18,
- au moins une pompe (340A ; 340B ; 340C ; 340D) et/ou une unité de traitement électronique (345A ; 345B) configurée(s) pour réguler une quantité et/ou une pression dudit fluide reçu dans l'espace intermédiaire primaire (230 ; 230B ; 630 ; 730 ; 830) de ladite prothèse de chambre cardiaque ou des prothèses de chambre cardiaque dudit ensemble de prothèses de chambre cardiaque de manière à modifier dynamiquement le volume de l'espace intermédiaire primaire (230 ; 230B ; 630 ; 730 ; 830) de ladite prothèse de chambre cardiaque ou des prothèses de chambre cardiaque dudit ensemble de prothèses de chambre cardiaque.
